# EUROPÄISCHE PATENTANMELDUNG

(11) **EP 0 778 271 A2**
(43) Veröffentlichungstag der Anmeldung: **11.06.1997**
(21) Anmeldenummer: 96119172.3
(22) Anmeldetag: 29.11.1996
(51) Int. Cl.: C07D 275/04, C07D 333/54, C07D 261/20, C07D 307/80, C07D 307/81, C07D 307/79, C07D 217/02, C07D 217/14, C07D 265/02, C07D 237/30, C07D 231/56, C07D 239/74, C07D 215/12, C07D 311/58, A61K 31/42, A61K 31/425, A61K 31/38, A61K 31/34, A61K 31/47, A61K 31/535, A61K 31/415, A61K 31/35, A61K 31/505

(54) **Aminoalkyl-substituierte benzo-heterocyclische Verbindungen**

(30) Priorität: 08.12.1995 CH 3480/95
(71) Anmelder: F. HOFFMANN-LA ROCHE AG, 4070 Basel (CH)
(72) Erfinder: Aebi, Johannes, 4051 Basle (CH); Lengsfeld, Hans, 4054 Basel (CH); Dehmlow, Henrietta, 79639 Grenzach-Wyhlen (DE); Morand, Olivier, 68220 Hegenheim (FR); Himber, Jacques, 68500 Guebwiller (FR); Schmid, Gérard, 4468 Kienberg (CH); Märki, Hans-Peter, 4059 Basle (CH); Ji, Yu-Hua, 4058 Basle (CH)
(74) Vertreter: Mahé, Jean

(57) **Zusammenfassung**

Aminoalkyl-substituierte benzo-heterocyclische Verbindungen der Formel 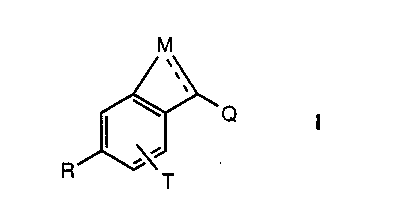 worin
- R: entweder eine Gruppe der Formel 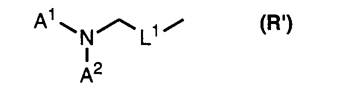
- T: H, Alkyl, Halogen, N(R²,R²¹), CONH₂, CN, NO₂, CF₃, OH oder Alkyl (O oder S),
- R² und R²¹: Alkyl oder H und
- Q: Cycloalkyl, gegebenenfalls substituiertes Phenyl oder gegebenenfalls durch OH substituiertes Alkyl Alkenyl oder ist,
oder worin eins von R und T Halogen oder H und das andere H, Alkyl, Halogen, N(R²,R²¹), CONH₂, CN, NO₂, CF₃, OH oder Alkyl (O oder S) und
- Q: eine Gruppe der Formel 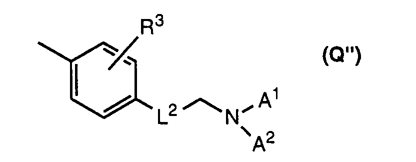
- A¹: Alkyl oder Alkenyl und
- A²: Cycloalkyl, Cycloalkyl-alkyl oder gegebenenfalls substituiertes Alkyl oder Alkenyl oder
A¹ zusammen mit A² gegebenenfalls substituiertes Alkylen oder Alkenylen,
- L¹: eine direkt oder über O, NH oder N(Alkyl oder Alkanoyl) an die Benzogruppe gebundene Gruppe L,
- L²: eine über O, NH oder N(Alkyl oder Alkanoyl) an die Phenylgruppe gebundene Gruppe L,
- L: Alkylen Alkenylen oder Cycloalkylen-alkylen,
die von M ausgehende gestrichelte Bindung fakultativ ist,
- M: eine 2- oder 3-gliedrige Gruppierung, die ein O- oder S-Atom oder eine Gruppe S(O), S(O)₂ oder C(O) und/oder bis zu zwei N-Atome oder Gruppen N(R⁶) und/oder bis zu zwei Gruppen C(R⁶) oder CH(R⁶) enthalten kann, jedoch insgesamt zumindest ein gegebenenfalls substituiertes Heteroatom enthält und
- R⁶: H oder Alkyl ist,
und Säureadditionssalze davon.

## Beschreibung

Die vorliegende Erfindung betrifft neue Aminoalkyl-substituierte benzo-heterocyclische Verbindungen, Verfahren zu deren Herstellung, pharmazeutische Präparate, die solche Verbindungen enthalten, sowie die Verwendung dieser Verbindungen bei der Herstellung pharmazeutischer Präparate.

Die Erfindung betrifft insbesondere die Aminoalkyl-substituierte benzo-heterocyclischen Verbindungen der Formel 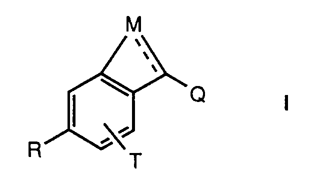 worin
- R: entweder eine Gruppe der Formel 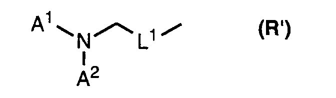
- T: H, Alkyl, Halogen, N(R²,R²¹), CONH₂, CN, NO₂, CF₃, OH oder Alkyl (O oder S),
- R² und R²¹: Alkyl oder H und
- Q: Cycloalkyl, durch R³ substituiertes Phenyl oder eine gegebenenfalls durch OH substituierte Alkenyl- oder Alkadienylgruppe mit bis zu 13 C-Atomen oder, falls M nicht durch ein O-Atom unterbrochen ist, dann Q auch gegebenenfalls durch OH substituiertes C₁₋₁₃-Alkyl sein kann,
oder worin eins von R und T Halogen oder H und das andere H, Alkyl, Halogen, N(R²,R²¹), CONH₂, CN, NO₂, CF₃, OH oder Alkyl (O oder S) und
- Q: eine Gruppe der Formel 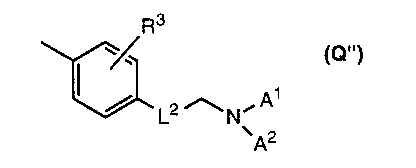
- A¹: Alkyl oder Alkenyl und
- A²: Cycloalkyl, Cycloalkyl-alkyl oder eine gegebenenfalls durch R⁴ substituierte Alkyl- oder Alkenylgruppe oder
A¹ zusammen mit A² eine gegebenenfalls durch R⁴ substituierte Alkylen- oder Alkenylengruppe A¹-A² mit bis zu 5 C-Atomen bildet,
- R⁴: an einem gesättigten C-Atom von A² oder von A¹-A² gebundenes OH oder Alkyl (O oder S), wobei ein durch R⁴ substituiertes C-Atom bzw. ein in A¹, A² oder A¹-A² enthaltenes ungesättigtes C-Atom in einer anderen als der α-Stellung zu N(A¹A²) gebunden sein soll,
- L¹: eine direkt oder über O, NH oder N(Alkyl oder Alkanoyl) an die Benzogruppe gebundene Gruppe L,
- L²: eine über O, NH oder N(Alkyl oder Alkanoyl) an die Phenylgruppe gebundene Gruppe L,
- L: eine Alkylen- oder Alkenylengruppe mit insgesamt bis zu 11 C-Atomen und zumindest 4 bzw. 3 C-Atomen zwischen den zwei freien Valenzen oder eine (C₃₋₆-Cycloalkylen)-alkylengruppe, die über die Cycloalkylengruppe an die Methylengruppe gebunden ist,
- R³ und R³¹: H, Alkyl, Halogen, N(R⁵,R⁵¹), CONH₂, CN, NO₂, CF₃, OH oder Alkyl (O oder S),
- R⁵ und R⁵¹: Alkyl oder H,
die von M ausgehende gestrichelte Bindung fakultativ ist,
- M: eine 2- oder 3-gliedrige Gruppierung, die ein O- oder S-Atom oder eine Gruppe S(O), S(O)₂ oder C(O) und/oder bis zu zwei N-Atome oder Gruppen N(R⁶) und/oder bis zu zwei Gruppen C(R⁶) oder CH(R⁶) enthalten kann, jedoch insgesamt zumindest ein gegebenenfalls substituiertes Heteroatom enthält und
- R⁶: H oder Alkyl ist,
und Säureadditionssalze davon.

Im Rahmen der vorliegenden Erfindung bezeichnen Ausdrücke, wie "Alkyl", "Alkenyl" und "Alkadienyl" allein oder in Kombination, wie in Cycloalkyl-alkyl, einbindige und wenn nicht anders angegeben, geradkettige oder verzweigte Gruppen mit bis zu 20, insbesondere bis zu 13, vorzugsweise bis zu 8 C-Atomen, bei Alkyl und Alkenyl bis zu 6, insbesondere bis zu 4 C-Atome. Beispiele für Alkyl sind Methyl, Aethyl, Propyl, Isopropyl, n-, s- und t-Butyl, Pentyl, Hexyl, Decyl und Dodecyl, für Alkanoyl: Formyl und Acetyl, für Alkenyl: Vinyl, Allyl, Propenyl, Butenyl, 3-Methyl-2-butenyl, 4-Methyl-3-pentenyl und Undodecenyl, für Alkadienyl: 4-Methyl-1,3-pentadienyl; 3,7-Dimethyl-2,6-octadienyl und 4,8-Dimethyl-3,7-nonadienyl. "Alkylen" und "Alkenylen" bezeichnen den weiter oben definierten einbindigen Alkyl- bzw. Alkenylgruppen entsprechende zweibindige Gruppen, wie Pentylen und 3-Methyl-pentylen; Propenylen und 2,6-Dimethyl-1-hexenylen. "Cycloalkyl" und "Cycloalkylen" allein oder in Kombination enthalten 3 bis 6 C-Atome, wie z.B. Cyclopropyl und Cyclohexyl bzw. Cyclopropylen.

Vorzugsweise steht A¹ für Methyl oder Allyl; A² für Methyl, Allyl, 2-Methylsulfanyl-äthyl oder Cyclopropyl; L¹ und L² für (CH₂)₅O, CH=CHCH₂O odere Cyclopropylen-methylenoxy; Q für Bromphenyl, Methyl, 4-Methyl-3-pentenyl, 2-Hydroxy-4-methyl-3-pentenyl, 4-Methyl-2,4-pentadienyl; R und T für H oder F; R³ für H, Br oder F und R⁶ für H oder Methyl.

Als pharmazeutisch annehmbare Säureadditionssalze kommen Salze der Verbindungen I mit anorganischen und organischen Säuren, wie HCl, HBr, H₂SO₄, HNO₃, Citronensäure, Essigsäure, Bernsteinsäure, Fumarsäure, Weinsäure, Methansulfonsäure und p-Toluolsulfonsäure, in Betracht.

Die Verbindungen der Formel I, die ein oder mehrere asymmetrische C-Atome enthalten, können als Enantiomere, als Diastereomere oder als Gemische davon, z.B. als Racemate, vorliegen.

Unter die Formel I fallende Verbindungen sind:
a) diejenigen der Formel 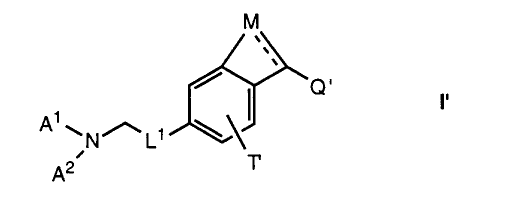 worin
   - T': H, Alkyl, Halogen, N(R²,R²¹), CONH₂, CN, NO₂, CF₃, OH oder Alkyl (O oder S),
   - Q': Cycloalkyl, durch R³ substituiertes Phenyl oder eine gegebenenfalls durch OH substituierte Alkenyl- oder Alkadienylgruppe mit bis zu 13 C-Atomen oder, falls M nicht durch ein O-Atom unterbrochen ist, dann Q auch gegebenenfalls durch OH substituiertes C₁₋₁₃-Alkyl sein kann,
   - L¹: eine direkt oder über O, NH oder N(Alkyl oder Alkanoyl) an die Benzogruppe gebundene Gruppe L ist und
   - A¹, A², L, M, R², R²¹ und R³: die gleiche Bedeutung wie in Anspruch 1 haben,
b) diejenigen der Formel 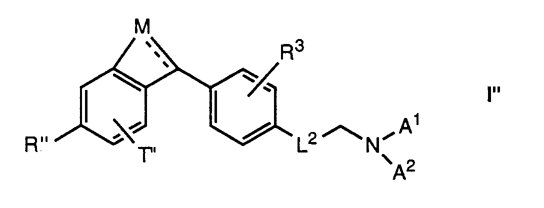 worin
   eins von R'' und T'' Halogen oder H und das andere H, Alkyl, Halogen, N(R²,R²¹), CONH₂, CN, NO₂, CF₃, OH oder Alkyl (O oder S),
   - L²: eine über O, NH oder N(Alkyl oder Alkanoyl) an die Phenylgruppe gebundene Gruppe L ist und
   - A¹, A², L, M, R², R²¹ und R³: die gleiche Bedeutung wie im Anspruch 1 haben.

Bevorzugt unter den erfindungsgemässen Verbindungen sind:
A) diejenigen der Formel 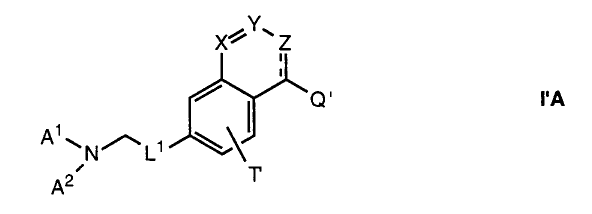 worin die Gruppierung X=Y-Z ein oder zwei N-Atome und zwei bzw. eine Gruppe CH oder C(Alkyl) enthält, insbesondere worin X=Y-Z eine Gruppierung CH=CH-N, CH=N-N, N=CH-N, N=CH-NH, N=CH-CH oder N=C(Alkyl)-CH, speziell N=C(CH₃)-CH ist,
B) diejenigen der Formel 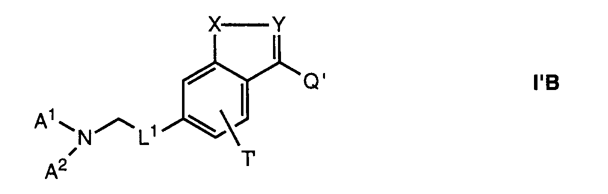 worin X ein O- oder S-Atom oder eine Gruppe SO₂, NH oder N(Alkyl), insbesondere N(CH₃), und Y ein N-Atom oder CH ist,
C) diejenigen der Formel 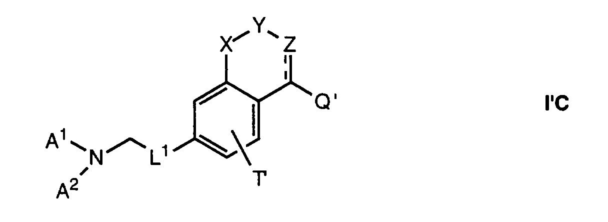 worin die Gruppierung X-Y-Z ein O-Atom oder eine Gruppe C(O) und/oder bis zu zwei N-Atome oder Gruppen N(R⁶) und/oder bis zu zwei Gruppen C(R⁶) oder CH(R⁶) enthalten kann, jedoch insgesamt ein oder zwei gegebenenfalls substituierte Heteroatome enthält, insbesondere worin X-Y-Z eine Gruppierung CH₂-O-N, O-CH₂-CH, CH₂-CH₂-N, N(R⁶)-C(O)-N, speziell N(CH₃)-C(O)-N oder CH₂-CH₂-N(R⁶), insbesondere CH₂-CH₂-NH oder CH₂-CH₂-N(CH₃) ist,
D) diejenigen der Formel 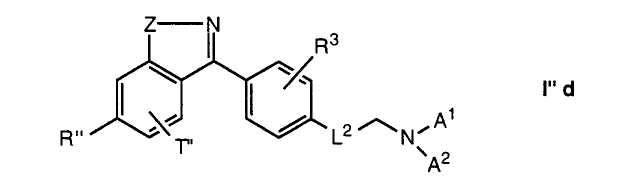 worin Z ein O- oder S-Atom oder eine Gruppe SO₂ oder N(Alkyl), insbesondere N-Methyl, oder worin Z zusammen mit N die Gruppe CH₂-O-N ist.

Bevorzugte Verbindungen der Formel I'A sind diejenigen, worin A¹ Alkyl, A² Alkenyl oder Cycloalkyl, T' Wasserstoff, Q' durch R³ substituiertes Phenyl und L¹ eine über ein O-Atom an die Benzogruppe gebundene Gruppe L, ist und/oder worin X=Y-Z eine Gruppierung CH=CH-N, CH=N-N, N=CH-N oder N=CH-CH, insbesondere worin A¹ Methyl, A² Allyl oder Cyclopropyl, L¹ n-Pentylenoxy oder Cyclopropylenmethylenoxy, T' Wasserstoff und Q' durch Brom substituiertes Phenyl ist, speziell die Verbindungen:
Allyl-[6- [1-(4-brom-phenyl)-isoquinolin-6-yloxy]-hexyl]-methyl-amin
[6-[1-(4-Brom-phenyl)-isoquinolin-6-yloxy]-hexyl]-cyclopropyl-methyl-amin,
Allyl-[6-[1-(4-brom-phenyl)-phthalazin-6-yloxy]-hexyl]-methyl-amin
Allyl-[6-[4-(4-brom-phenyl)-quinazolin-7-yloxy]-hexyl]-methyl-amin
Allyl-[6-[4-(4-brom-phenyl)-quinolin-7-yloxy]-hexyl]-methyl-amin
(1RS,2RS)-[2-[1-(4-Brom-phenyl)-isoquinolin-6-yloxymethyl]-cyclopropyl-methyl]-cyclopropyl-methyl-amin,
sowie die Verbindungen:
(E)-Allyl-[4-[1-(4-brom-phenyl)-isoquinolin-6-yloxy]-but-2-enyl]-methyl-amin
(E)-Allyl-[4-[1-(4-brom-phenyl)-phthalazin-6-yloxy]-but-2-enyl]-methyl-amin
Allyl-[6-[4-(4-brom-phenyl)-2-methyl-quinolin-7-yloxy]-hexyl]-methyl-amin.

Bevorzugte Verbindungen der Formel I'B sind diejenigen, worin A¹ Alkyl, A² Alkenyl, Cycloalkyl oder Alkyl-S-alkyl, L¹ eine über ein O-Atom an die Benzogruppe gebundene Alkylen- oder Alkenylengruppe mit insgesamt bis zu 11 C-Atomen und zumindest 4 bzw. 3 C-Atomen zwischen den zwei freien Valenzen, T' Wasserstoff oder Halogen und Q' durch R³ substituiertes Phenyl oder eine Alkyl- oder gegebenenfalls durch OH substituierte Alkenyl- oder Alkadienylgruppe mit bis zu 13 C-Atomen ist, und/oder
worin X-Y eine Gruppe S-CH, O-N, S-N, NH-N oder N(CH₃)-N, insbesondere worin A¹ Methyl, A² Allyl, Cyclopropyl oder Methylsulfanyl-ethyl, L¹ n-Pentylenoxy oder n-Propenylenoxy, Q' Bromphenyl oder 4-Methyl-pent-3-enyl und T' Wasserstoff oder Fluor ist, speziell die Verbindungen:
Allyl-[6-[3-(4-brom-phenyl)-benzo[d]isothiazol-6-yloxy]-hexyl]-methyl-amin
6-[3-(4-Brom-phenyl)-benzo[d]isothiazol-6-yloxy]-hexyl]-methyl-(2-methylsulfanyl-ethyl)-amin
Allyl-[6-[3-(4-brom-phenyl)-benzo[b]thiophen-6-yloxy]-hexyl]-methyl-amin
(E)-Allyl-methyl-[4-[3-(4-methyl-pent-3-enyl)-benzo[d]isothiazol-6-yloxy]-but-2-enyl]-amin
(E)-Allyl-[4-[3-(4-brom-phenyl)-benzo[d]isoxazol-6-yloxy]-but-2-enyl-methyl-amin
(E)-Allyl-[4-[3-(4-brom-phenyl)-5-fluor-1-methyl-1H-indazol-6-yloxy]-but-2-enyl]-methyl-amin
Allyl-methyl-[6-[1-methyl-3-(4-methyl-pent-3-enyl)-1H-indazol-6-yloxy]-hexyl]-amin
(E)-Allyl-methyl-[4-[1-methyl-3-(4-methyl-pent-3-enyl)-1H-indazol-6-yloxy]-but-2-enyl]-amin
[6-[3-(4-Brom-phenyl)-1-methyl-1H-indazol-6-yloxy]-hexyl]-cyclopropyl-methyl-amin
Allyl-[6-[3-(4-brom-phenyl)-1H-indazol-6-yloxy]-hexyl]-methyl-amin,
sowie die Verbindungen:
(E)-Allyl-[4-[3-(4-brom-phenyl)-benzo[d]isothiazol-6-yloxy]-but-2-enyl]-methyl-amin
(E)-[4-[3-(4-Brom-phenyl)-benzo[d]isothiazol-6-yloxy]-but-2-enyl]-methyl-(2-methylsulfanyl-ethyl)-amin
Allyl-methyl-[6-(3-methyl-benzo[d]isothiazol-6-yloxy)-hexyl]-amin
(E)-Allyl-methyl-[4-(3-methyl-benzo[d]isothiazol-6-yloxy)-but-2-enyl]-amin
(E)-Allyl-[4-[3-(4-brom-phenyl)-benzo[b]thiopen-6-yloxy]-but-2-enyl]-methyl-amin
Allyl-methyl-[6-[3-(4-methyl-pent-3-enyl)-benzo[d]isothiazol-6-yloxy]-hexyl-amin
(E)-Allyl-methyl-[6-[3-(4-methyl-penta-1,3-dienyl)-benzo[d]isothiazol-6-yloxy]-hexyl]-amin
(RS)-1-[6-[6-(Allyl-methyl-amino)-hexyloxy]-benzo[d]isothiazol-3-yl]-4-methyl-pent-3-en-2-ol
(E)-(RS)-1-[6-[4-(Allyl-methyl-amino)-but-2-enyloxy]-benzo[d]isothiazol-3-yl]-4-methyl-pent-3-en-2-ol
Allyl-methyl-[(E)-4-[3-[(E)-4-methyl-penta-1,3-dienyl]-benzo[d]isothiazol-6-yloxy]-but-2-enyl]-amin
7:3 Gemisch von (E)-Allyl-methyl-[6-[3-(4-methyl-penta-2,4-dienyl)-benzo[d]isothiazol-6-yloxy]-hexyl]-amin und (E)-Allyl-methyl-[6-[3-(4-methyl-penta-1,3-dienyl)-benzo[d]isothiazol-6-yloxy]-hexyl]-amin
Allyl-[6-[4-(6-brom-1,1-dioxo-benzo[d]isothiazol-3-yl)-phenoxy]-hexyl]-methyl-amin
Allyl-[6-[3-(4-brom-phenyl)-benzo[d]isoxazol-6-yloxy]-hexyl]-methyl-amin
[6-[3-(4-Brom-phenyl)-benzo[d]isoxazol-6-yloxy]-hexyl]-cyclopropyl-methyl-amin
(E)-Allyl-methyl-[4-[3-(4-methyl-pent-3-enyl)-benzo[d]isoxazol-6-yloxy]-but-2-enyl]-amin
(E)-Allyl-[4-[3-(4-brom-phenyl)-benzofuran-6-yloxy]-but-2-enyl]-methyl-amin
Allyl-[6-[3-(4-brom-phenyl)-benzofuran-6-yloxy]-hexyl]-methyl-amin
Allyl-[6-[3-(4-brom-phenyl)-1-methyl-1H-indazol-6-yloxy]-hexyl]-methyl-amin
(E)-Allyl-[4-[3-(4-brom-phenyl)-1-methyl-1H-indazol-6-yloxy]-but-2-enyl]-methyl-amin
Allyl-[6-(1,3-dimethyl-1H-indazol-6-yloxy)-hexyl]-methyl-amin
(E)-Allyl-[4-(1,3-dimethyl-1H-indazol-6-yloxy)-but-2-enyl]-methyl-amin
Cyclopropyl-methyl-[6-[3-(4-methyl-pent-3-enyl)-1H-indazol-6-yloxy]-hexyl]-amin.

Bevorzugte Verbindungen der Formel I'C sind diejenigen, worin A¹ Alkyl, A² Alkenyl, L¹ eine über ein O-Atom an die Benzogruppe gebundene Alkylen- oder Alkenylengruppe mit insgesamt bis zu 11 C-Atomen und zumindest 4 bzw. 3 C-Atomen zwischen den zwei freien Valenzen, T' Wasserstoff und Q' durch R³ substituiertes Phenyl ist, und/oder
worin X-Y-Z eine Gruppierung CH₂-O-N, CH₂-CH₂-N oder O-CH₂-CH, insbesondere worin A¹ Methyl, A² Allyl, L¹ n-Pentylenoxy oder n-Propenylenoxy, T' Wasserstoff und Q' Bromphenyl ist, speziell die Verbindungen:
(E)-Allyl-[4-[1-(4-brom-phenyl)-3,4-dihydro-isoquinolin-6-yloxy]-but-2-enyl]-methyl-amin
(E)-Allyl-[4-[4-(4-brom-phenyl)-1H-benzo[d][1,2]oxazin-7-yloxy]-but-2-enyl]-methyl-amin
Allyl-[6-[4-(4-brom-phenyl)-2H-chromen-7-yloxy]-hexyl]-methyl-amin,
sowie die Verbindungen:
Allyl-[6-[1-(4-brom-phenyl)-3,4-dihydro-isoquinolin-6-yloxy]-hexyl]-methyl-amin
(RS)-Allyl-[6-[1-(4-brom-phenyl)-1,2,3,4-tetrahydro-isoquinolin-6-yloxy]-hexyl]-methyl-amin
(RS)-Allyl-[6-[1-(4-brom-phenyl)-2-methyl-1,2,3,4-tetrahydro-isoquinolin-6-yloxy]-hexyl]-methyl-amin
Allyl-6-[4-(4-brom-phenyl)-1H-benzo[d][1,2]oxazin-7-yloxy]-hexyl-methyl-amin
7-[6-(Allyl-methyl-amino)-hexyloxy]-4-(4-brom-phenyl)-1-methyl-1H-quinazolin-2-on.

Bevorzugte Verbindungen der Formel I''d sind diejenigen, worin A¹ Alkyl, A² Alkenyl, L² über ein O-Atom an die Phenylgruppe gebundene Alkylen- oder Alkenylengruppe mit insgesamt bis zu 11 C-Atomen und zumindest 4 bzw. 3 C-Atomen zwischen den freien Valenzen, R³ Wasserstoff und R'' und T'' Wasserstoff oder Halogen sind,
und/oder worin Z ein O- oder S-Atom oder eine Gruppe SO₂ oder N-Methyl oder worin Z zusammen mit N die Gruppe CH₂-O-N, insbesondere worin A¹ Methyl, A² Allyl, L² n-Pentylenoxy oder n-Propylenoxy, R³ Wasserstoff oder Fluor, R'' Wasserstoff oder Brom und T'' Wasserstoff oder Fluor ist, speziell die Verbindungen:
Allyl-[6-[4-(6-brom-benzo[d]isothiazol-3-yl)-phenoxy]-hexyl]-methyl-amin
(E)-Allyl-[4-[4-(6-brom-benzo[d]isothiazol-3-yl)-phenoxy]-but-2-enyl]-methyl-amin
Allyl-[6-[4-(6-brom-1,1-dioxo-benzo[d]isothiazol-3-yl)-phenoxy]-hexyl]-methyl-amin
Allyl-[6-[4-(6-brom-benzo[d]isoxazol-3-yl)-phenoxy]-hexyl-methyl-amin
(E)-Allyl-[4-[4-(6-brom-benzo[d]isoxazol-3-yl)-phenoxy]-but-2-enyl]-methyl-amin
Allyl-[6-[4-(7-brom-1H-benzo[d][1,2]oxazin-4-yl)-phenoxy]-hexyl]-methyl-amin
(E)-Allyl-[4-[4-(7-brom-1H-benzo[d][1,2]oxazin-4-yl)-phenoxy]-but-2-enyl-methyl-amin
(E)-Allyl-[4-[2-fluor-4-(4-fluor-1-methyl-1H-indazol-3-yl)-phenoxy]-but-2-enyl]-methyl-amin
Allyl-[6-[4-(6-brom-1-methyl-1H-indazol-3-yl)-phenoxy]-hexyl]-methyl-amin.

Die Erfindung betrifft ferner ein Verfahren zur Herstellung der Verbindungen der Formel I. Dieses ist dadurch gekennzeichnet, dass man
a) ein Bromid der Formel 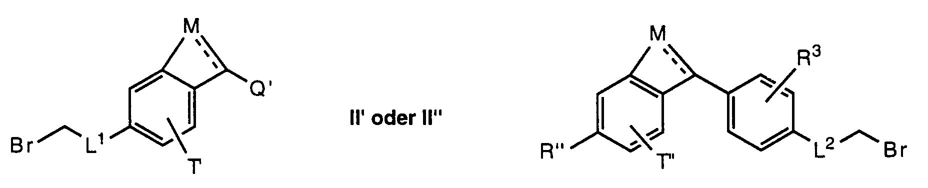 mit einem Amin HN(A^{1,}A²) umsetzt,
b) ein Keton der Formel 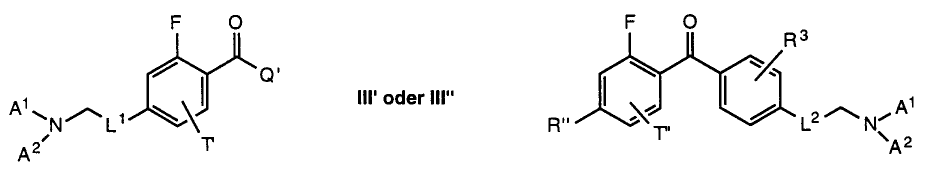 mittels H₂NNH-R⁶ cyclisiert,
c) ein Oxim der Formel 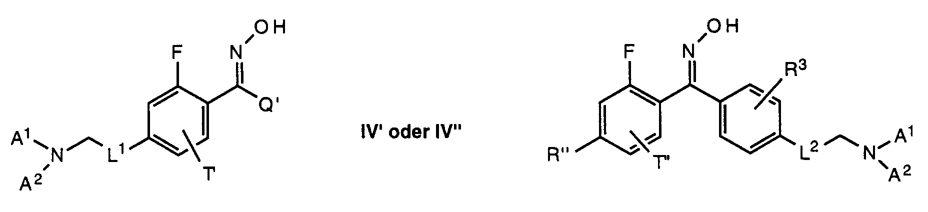 cyclisiert,
d) ein Aldehyd der Formel 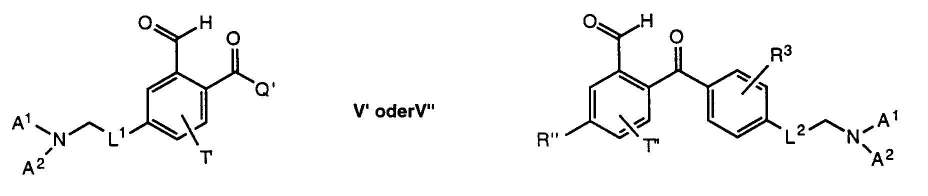 mittels Hydrazin cyclisiert,
e) ein Amin der Formel 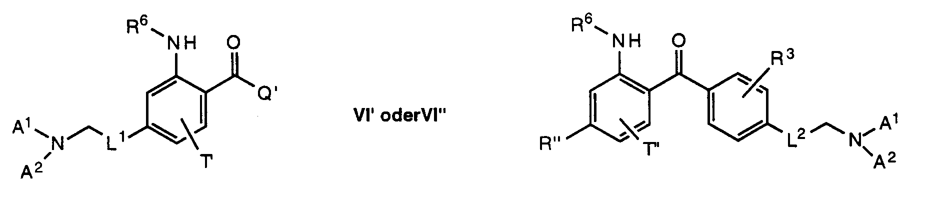 cyclisiert,
f) ein Phenol der Formel 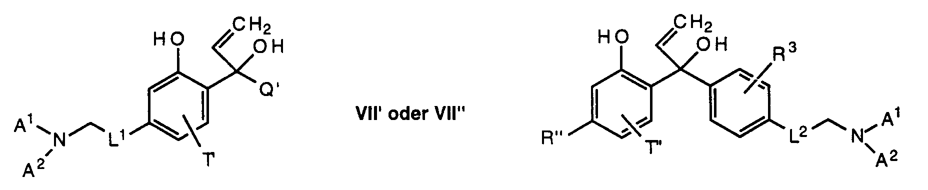 oder ein Anilin der Formel 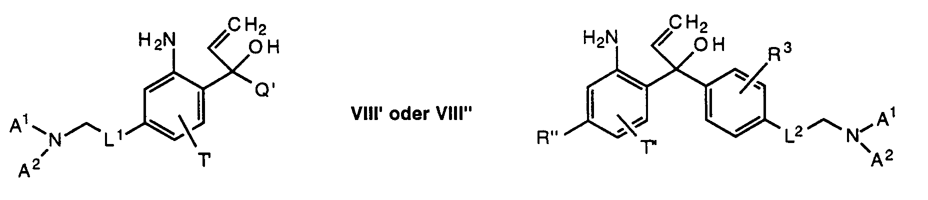 cyclisiert,
g) ein Phenol der Formel 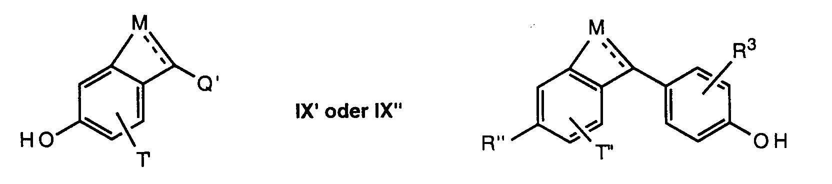 mit einem Aminoalkohol (A¹,A²)NCH₂-L-OH umsetzt,
h) gewünschtenfalls eine in einer Verbindung der Formel I enthaltene reaktionsfähige Gruppe funktionell abwandelt und
i) gewünschtenfalls ein Amin der Formel I in ein physiologisch verträgliches Säureadditionssalz oder ein Säureadditionssalz einer Verbindung der Formel I in das Amin der Formel I überführt.

Dieses Verfahren und dasjenige zur Herstellung der Ausgangsverbindungen der Formeln II' und II'' bis IX' und IX'' kann man in an sich bekannter Weise durchführen. So wird die Reaktion a) des Bromids II' oder II'' mit dem Amin HN(A¹,A²) in Dimethylacetamid (DMA) oder Dimethylformamid (DMF) oder in Aceton in Gegenwart von K₂CO₃ durchgeführt.

Bromide II' (oder II''), worin L¹ (oder L²) eine über O gebundene Gruppe L ist, erhält man durch Reaktion eines Dibromids BrCH₂-L-Br mit einem entsprechenden Phenol der Formel IX' oder IX''. Ein solches Phenol wird aus dem entsprechenden Methyläther und letzteres durch Cyclisierung eines Methyläthers der Formel 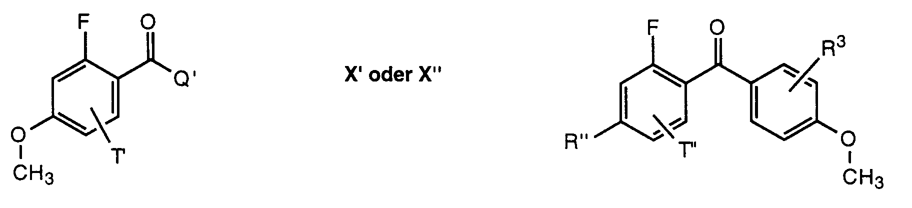 hergestellt. Diese Cyclisierung kann dadurch bewerkstelligt werden, dass man das Fluorid X 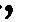 oder X' 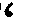 mittels Benzylmercaptan in Gegenwart von Kalium-tert.butylat in THF umsetzt, den erhaltenen Benzylthioäther in Methylenchlorid mit Sulfurylchlorid versetzt und die erhaltene Verbindung in THF mit einer Lösung von Ammoniak in Aethanol cyclisiert.

In einer Variante wird ein Methyläther der Formel 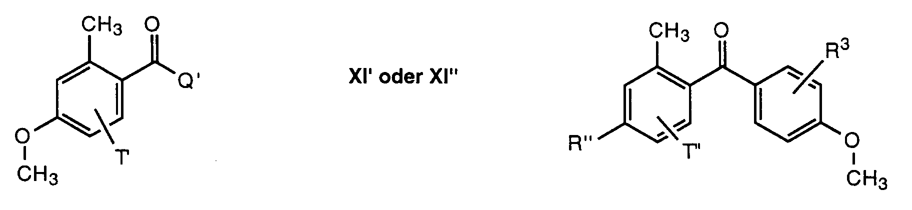 via einer Verbindung der Formel 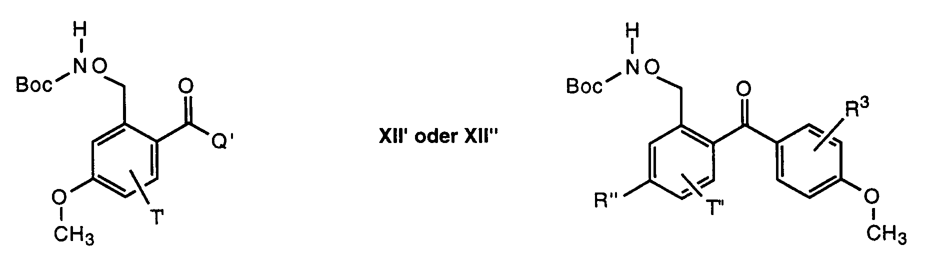 in das Phenol der Formel 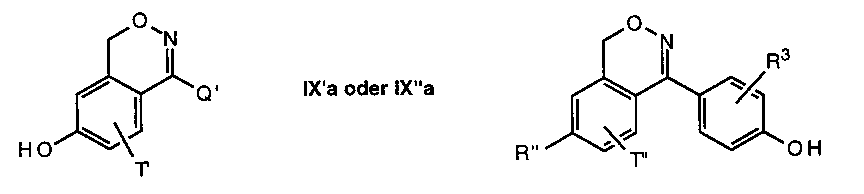 überführt.

Die Cyclisierung b) eines Ketons III' oder III'' mittels gegebenenfalls alkyliertem Hydrazin der Formel H₂NNH-R⁶ wird in DMA in Gegenwart von Kaliumcarbonat bei einer Temperatur bis zu 150°C, vorzugsweise etwa 120°C, durchgeführt. Sie führt zu Verbindungen der Formel I, worin M eine Gruppe N(R⁶)-N ist.

Ketone III' und III'' erhält man ausgehend von dem entsprechenden Methyläther X' bzw. X'' via den entsprechenden Phenolen, z.B. wie in den nachfolgenden Beispielen A und B beschrieben.

Die Cyclisierung c) eines Oxims IV' oder IV'' erfolgt in DMA in Gegenwart von Kaliumcarbonat unter Erhitzen bei einer Temperatur bis zu 160°C, vorzugsweise etwa 130°C. Sie führt zu Verbindungen I, worin M die Gruppe ON ist.

Oxime IV' und IV'' erhält man durch Reaktion der Ketone III' bzw. III'' mit NH₂OH, HCl in Gegenwart von Natriumacetat in Aethanol unter Erhitzen bei einer Temperatur von etwa 90°C.

Ein Aldehyd V' oder V'' lässt sich mittels Hydrazinhydrat in Aethanol zu einer Verbindung der Formel I, worin M die Gruppe CH=N-N ist, cyclisieren. Aldehyde V' und V'' erhält man ausgehend von entsprechenden Verbindungender obigen Formeln XI' bzw. XI'' via die Dibromide der Formeln 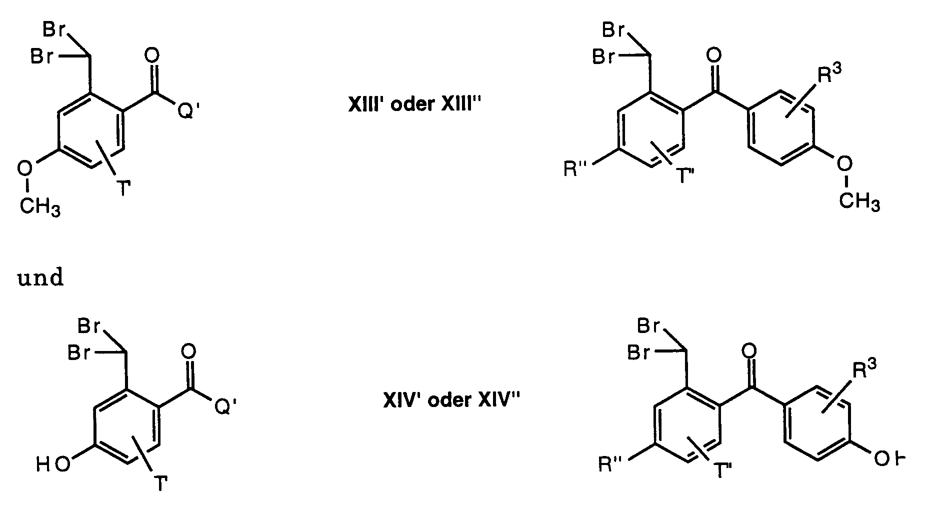 und den Aldehyden der Formeln

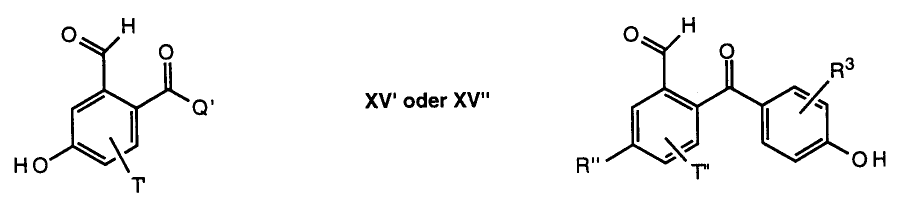

Die Cyclisierung e) eines Amins der Formel VI' oder VI'' führt zu Verbindungen der Formel I, worin M für N=CH-NH, N(R⁶)-C(O)-N oder N=C(R⁶)-CH steht, je nachdem ob man
1) ein Amin VI' oder VI'', worin R⁶ für H steht, mit Ameisensäure und Formamid bei erhöhter Tempratur,
2) ein Amin VI' oder VI'', worin R⁶ Alkyl ist, mit Chlorsulfonylisocyanat in Methylenchlorid unter Abkühlung oder
3) ein Amin VI' oder VI'', worin R⁶ für H steht, in Essigsäure und Schwefelsäure unter Erhitzen mit einem Keton R⁶-C(O)-CH₃ umsetzt.

Die Amine VI 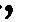 und VI 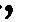 ' erhält man ausgehend von den Verbindungen III 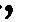 bzw. III' 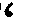 . Das in letzteren Verbindungen enthaltene Fluoratom wird entweder durch Reaktion mit Methoxybenzylamin in Gegenwart von einer Base, wie Kaliumcarbonat, in Toluol und dann mit Trifluoressigsäure in eine freie Aminogruppe, oder durch Reaktion in DMA mit einem Alkylamin R⁶-NH₂ in Ethanol in eine Alkylamingruppe R⁶-NH umgewandelt.

Die Cyclisierung eines Phenols VII' (oder VII'') bzw. eines Anilins VIII' (oder VIII'') führt zu einer Verbindung I, worin M eine Gruppe O-CH-CH bzw. N=CH-CH ist. Ein Phenol VII' oder VII'' wird in einem Lösungsmittel, wie o-Xylol, bei erhöhter Temperatur vorzugsweise beim Siedepunkt der Reaktionslösung am Wasserabscheider cyclisiert. Die Cyclisierung des Anilins VIII' oder VIII'' erfolgt in einem Lösungsmittel, wie Methylenchlorid, in Gegenwart von Pyridiniumchlorochromat unter Abkühlung, vorzugsweise auf etwa 0°C.

Phenole VII' und VII'' erhält man durch Reaktion der entsprechenden Verbindungen der Formeln 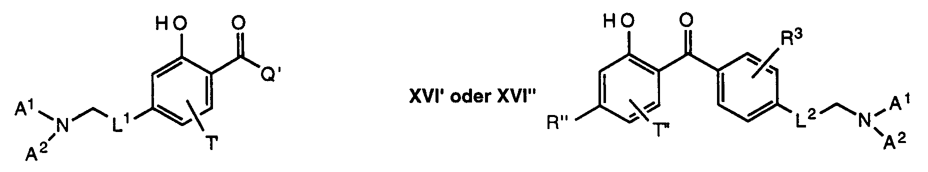 mit Vinylmagnesiumchlorid in THF/Diäthyläther bei etwa 0°C. Analog erhält man die Aniline VIII' und VIII'' aus den entsprechenden Verbindungen der Formeln VI' und VI'', worin R⁶ für H steht. Die Phenole XVI' und XVI'' erhält man ausgehend von den entsprechenden Fluoriden III' und III'' zunächst durch Reaktion mit Natriummethanolat und anschliessend Spaltung der entstandenen Methyläther mittels Essigsäure/Bromwasserstoffsäure.

Die Umsetzung g) wird in einer Lösung von Triphenylphosphin, Phenol der Formel IX' oder IX'' und Aminoalkohol der Formel (A¹,A²)NCH₂-L-OH in THF mittels Diäthylazodicarboxylat bewerkstelligt.

Als funktionelle Abwandlungen h) einer in einer Verbindung der Formel I enthaltenen reaktionsfähigen Gruppe können folgende genannt werden:
1) Durch Deprotonieren einer Verbindung der Formel I', worin Q' Methyl ist, zunächst mit Lithiumdiisopropylamid in THF bei -78°C und dann bei dieser Temperatur mit einem Bromid BrCH₂-R⁷ oder Aldehyd HC(O)- R⁷ worin R⁷ eine Alkyl-, Alkenyl- oder Alkadienylgruppe mit bis zu 12 C-Atomen ist, erhält man die entsprechende Verbindung I', worin Q' für CH₂CH₂-R⁷ bzw. CH₂CH(OH)-R⁷ steht.
2) Durch Hydrierung, z.B. mit Natriumborhydrid, kann man in einer Verbindung I, worin M eine Gruppe CH₂-CH₂-N ist, diese Gruppe in die Gruppe CH₂-CH₂-NH umwandeln.
3) Eine Verbindung I, worin M die Gruppe CH₂-CH₂-NH kann in Gegenwart von NaH₂PO₃ in Dioxan mittels Formaldehyd zur entsprechenden Verbindung I, worin M die Gruppe CH₂-CH₂-N(CH₃) ist, methyliert werden.

In den nachfolgenden Beispielen A bis B ist die Herstellung von einigen der weiter oben erwähnten Ausgangsmaterialien und Zwischenprodukten beschrieben.

Die Erfindung betrifft die Verbindungen der Formel I und deren Salze zur Verwendung als therapeutische Wirkstoffe, ferner antimykotisch wirksame und Cholesterin senkende Arzneimittel, enthaltend eine Verbindung der Formel I oder ein Salz davon als Wirkstoff, gegebenenfalls neben einem therapeutisch inerten Träger, sowie die Verwendung der Verbindungen der Formel I und der Salze davon für die Herstellung von den oben genannten Arzneimittel.

Cholesterin ist ein Hauptbestandteil der atherosklerotischen Plaques. Der Zusammenhang zwischen koronarer Herzkrankheit (KHK) und hohen LDL-Cholesterinkonzentrationen im Plasma (LDL = Lipoproteine niedriger Dichte) und der therapeutische Nutzen der Senkung erhöhter LDL-Konzentrationen werden heute allgemein anerkannt (Gotto et al., Circulation 81, 1990, 1721-1733; Stein et al., Nutr. Metab. Cardiovasc. Dis. 2, 1992, 113-156). Atherosklerotische Plaques können wachsen und zum Verschluss von Blutgefässen führen, was eine Ischämie oder einen Infarkt zur Folge hat. Studien zur Primärprophylaxe haben ergeben, dass eine Senkung der LDL-Konzentrationen im Plasma die Häufigkeit der nichttödlichen Anfälle von KHK verringert, während die Gesamtsterblichkeit unverändert bleibt. Die Senkung des LDL-Cholesterinspielgels im Plasma von Patienten mit klinisch bestätigter KHK (sekundäre Intervention) reduziert die KHK-bedingte Sterblichkeit und Morbidität; die Metaanalyse verschiedener Studien zeigt, dass diese Abnahme proportional ist zur Verringerung des LDL-Cholesterins.

Der klinische Nutzen der Cholesterinsenkung ist für Patienten mit bestätigter KHK noch grösser als für asymptomatische Personen mit Hypercholesterinämie. Für die meisten Patienten, die einen Myokardinfarkt überlebt haben, sowie für die Patienten, die an Angina pectoris oder einer anderen atherosklerotischen Erkrankung leiden, ist die Behandlung mit einem Lipidsenker zu empfehlen, wobei eine Konzentration des LDL-Cholesterins von 2,6 mmol/l angestrebt werden sollte.

Für die üblichen Standardtherapien werden Präparate, wie gallensäuresequestrierende Präparate, Fibrate, Nicotinsäure, Probucol sowie die Statine (HMG-CoA-Reduktasehemmer), wie Lovastatin und Simvastatin, verwendet. Für an KHK leidende Patienten, die hohe LDL-Cholesterinspiegel aufweisen und bei denen sich der angestrebte Wert von 2,5 bis 3,0 mmol/l mit Statinen nicht erreichen lässt, wäre ein neues cholesterinsenkendes Medikament von beträchtlichem Nutzen.

Ferner weisen die Statine unerwünschte Nebenwirkungen auf. Sie hemmen die Cholesterinproduktion in einer frühen Phase der Synthesekaskade, wodurch auch die Bildung nicht-steriner Isoprenoide gehemmt wird. Letztere sind für Zellfunktionen unerlässlich. Die Regulation des Zellcyclus, die Modifikation von Eiweissen und der Transport von Elektronen in der Atmungskette können daher durch Statine beeinträchtigt werden.

Aus diesem Grund wurden mehrere Versuche unternommen, plasmacholesterinsenkende Medikamente zu finden, welche die Cholesterinsynthese einerseits nach der Stufe des Farnesyl-Pyrophosphates hemmen, damit die Bildung nicht-steriner Isoprenoide nicht gehemmt wird, und anderseits vor dem Lanosterin, damit es nicht zur Akkumulation von Sterinzwischenprodukten kommt. Zu diesen Substanzen gehören die in der europäischen Patentanmeldung Nr. 636 367 beschriebenen Verbindungen, die die 2,3-Oxidosqualen-lanosterincyclase (OSC) hemmen und das Gesamtcholesterin im Plasma senken.

Die vorliegenden Verbindungen der Formel I hemmen die Cholesterinsynthese und verringern das Gesamtcholesterin im Plasma. Man kann sie daher bei der Therapie und Prophylaxe von Hypercholesterinämie, Hyperlipämie und Arteriosklerose verwenden. Sie besitzen gegenüber bekannten Verbindungen eine bessere Verträglichkeit und stärkere Wirksamkeit. Ferner können sie in der Therapie von Mykosen und hyperproliferativen Erkrankungen Verwendung finden. Zum Beweis der Wirksamkeit der Verbindungen der Formel I und ihrer Salze wurden folgende Versuche durchgeführt.

### Hemmung der humanlebermikrosomalen 2,3-Oxidosqualen-lanosterincyclase(OSC)

Lebermikrosomen eines gesunden Spenders wurden in Natriumphosphatpuffer(pH 7.4) zubereitet. Die OSC-Aktivität wurde im selben Puffer, der zusätzlich 1 mM EDTA und 1 mM Dithiothreitol enthielt, gemessen. Die Mikrosomen wurden in kaltem Phosphatpuffer auf 0.8 mg/ml Protein verdünnt. Trockenes [¹⁴C]R,S-Monooxidosqualen (MOS; 12.8 mCi/mmol) wurde mit Aethanol auf 20 nCi/µl verdünnt und mit Phosphatpuffer-1% BSA (Bovine Serum Albumin) gemischt. Eine Stammlösung von 1 mM Testsubstanz in DMSO wurde mit Phosphatpuffer-1% BSA auf die gewünschte Konzentration verdünnt. Es wurden 40 µl Mikrosomen mit 20 µl der Lösung der Testsubstanz gemischt und die Reaktion anschliessend mit 20 µl der [¹⁴C]R,S-MOS-Lösung gestartet. Die Endbedingungen waren: 0,4 mg/ml mikrosomalen Proteine und 30 µl [¹⁴C]R,S-MOS in Phosphatpuffer, pH 7.4, enthaltend 0.5% Albumin, DMSO <0.1% und Aethanol <2%, in einem Gesamtvolumen von 80 µl.

Nach 1 Stunde bei 37°C wurde die Reaktion gestoppt durch Zugabe von 0,6 ml 10% KOH-Methanol, 0,7 ml Wasser und 0,1 ml Hexan:Aether (1:1, v/v), die je 25 µg nicht-radioaktives MOS und Lanosterin als Träger enthielten. Nach Schütteln wurde jedem Röhrchen 1 ml Hexan:Aether (1:1, v/v) zugegeben, nochmals geschüttelt und dann zentrifugiert. Die obere Phase wurde in ein Glasröhrchen transferiert, die untere Phase nochmals mit Hexan:Aether extrahiert, und mit dem ersten Extrakt vereint. Der gesamte Extrakt wurde mit Stickstoff zur Trockene eingedampft, in 50 µl Hexan: Aether suspendiert und auf eine Kieselgelplatte aufgetragen. Die chromatographische Trennung erfolgte in Hexan:Aether (1:1, v/v) als Eluens. Die Rf-Werte für das Substrat MOS und das Produkt Lanosterin betrugen 0,91 bzw. 0,54. Nach dem Trocknen wurden radioaktives MOS und Lanosterin auf der Kieselgelplatte beobachtet. Aus den radioaktiven Banden wurde das Verhältnis von MOS zu Lanosterin ermittelt, um die Hemmung der OSC zu bestimmen.

Der Test wurde einerseits mit einer konstanten Konzentration der Testsubstanz von 100 nM durchgeführt und die prozentuale Hemmung der OSC gegenüber Kontrollen berechnet. Ferner wurde der Test mit verschiedenen Konzentrationen der Testsubstanz durchgeführt und anschliessend der IC₅₀-Wert berechnet, d.h. die Konzentration, die erforderlich ist, um die Umwandlung von MOS in Lanosterin auf 50% des Kontrollwertes zu verringern. Die Resultate sind in der nachstehenden Tabelle angegeben:

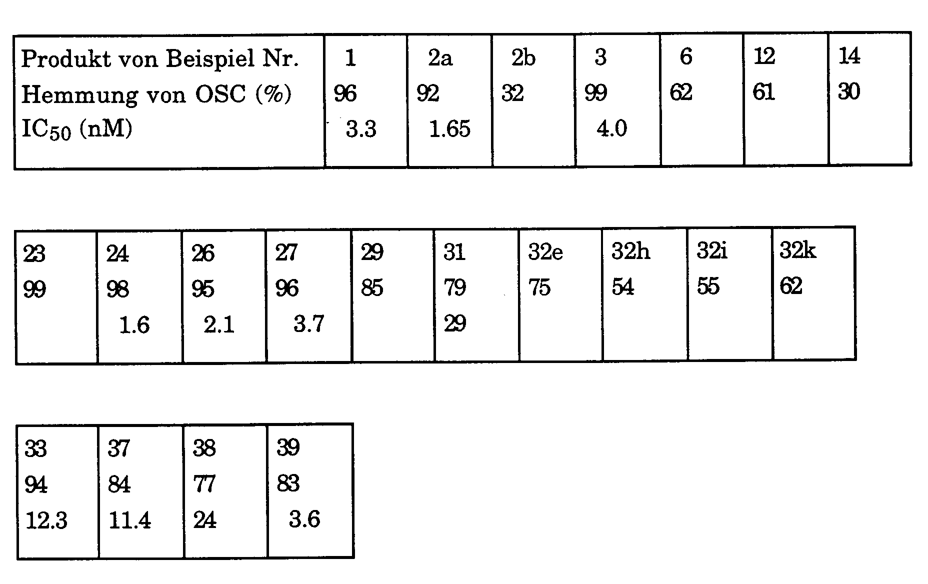

### Cholesterinsenkung in fettgefütterten Hamstern

Männliche Goldhamster in Einzelhaltung wurden während 7 Tagen mit Kokosnussraspel haltiger Diät (40 cal.% Fett) vorbehandelt. Die Tiere wurden dann in Gruppen von 5 Tieren eingeteilt. Während der Behandlung wurden die Tiere auf derselben Diät gehalten. Jede Testsubstanz wurde zuerst in 9 ml Wasser homogenisiert und anschliessend mit der gemahlenen Diät vermischt. Die Kontrollen erhielten lediglich mit Wasser angeteigtes Futter. Die Tiere wurden mit einer Dosis der Testsubstanz von 200 µmol (etwa 70-120 mg/kg/Tag) während 10 Tagen behandelt. Blutproben (200 µl) wurden unter leichter Anästhesie via Jugular-Vene genommen, und zwar je am letzten Tag der Vorbehandlung und einen Tag nach der letzten Verabreichung der Testsubstanz. Die Konzentration des Cholesterins im Plasma wurde mit einer kolorimetrischen Enzymmethode bestimmt. Die Plasmalipoproteine wurden durch Ausschlusschromatographie (Hennes et al., Science Tools, 36, 1992, 10-12) getrennt. Das Gesamtcholesterin wurde in jeder Fraktion mit einer fluorometrischen Enzymmmethode bestimmt (Gamble et al., J. Lipid Res., 19, 1978, 1068-1071), um die Menge des Cholesterins in den LDL- und HDL-Fraktionen zu berechnen. Für die Produkte der Beispiele 8a und 12 ist die Wirkung auf das Plasmacholesterin sowie das LDL- und HDL-Cholesterin, ausgedrückt in Prozent der Kontroll-Tiere, in der nachstehenden Tabelle wiedergegeben.

| Beispiel | 1 | 23 |
|---|---|---|
| Gesamtcholesterin | -33% | -15% |
| LDL-Cholesterin | -36% | -23% |
| HDL-Cholesterin | -27% | - 2% |

Wie bereits erwähnt besitzen die Verbindungen der Formel I und ihre pharmazeutisch annehmbaren Säureadditionssalze ausserdem wertvolle antifungale Eigenschaften. Sie sind wirksam gegen eine Vielzahl von pathogenen Pilzen, die topische und systemische Infektionen hervorrufen, wie Candida albicans, Cryptococcus neoformans und Aspergillus fumigatus.

### Antifungale Aktivität in vitro

Die Verbindungen wurden auf antifungale Aktivität gegen Candida albicans, Cryptococcus neoformans und Aspergillus fumigatus mittels einer Mikroverdünnungsmethode auf Mikrotiterplatten (96 Löcher pro Platte) getestet. Mit 1% Glukose und 0,25% di-Kaliumphosphat angereicherte Hefe wurde für die drei Pilzstämme verwendet. Die Pilzzellen wurden mit 3x10⁴ cfu (colony forming unit) in 1 ml Medium pro Loch angeimpft. Das Medium enthielt steigende Konzentrationen des Testsubstanz. Nach 24 bzw. 48 Stunden Inkubation bei 27°C wurde die Trübung in jedem Loch durch einen Mikrotiterplatten-Leser gemessen. Die Hemmung des Wachstums wurde im Vergleich zur Kontrolle (ohne Testsubstanz) berechnet. Die in der nachstehenden Tabelle angegebenen IC₅₀-Werte Konzentration ist die der Testsubstanz, bei der das Wachstum um 50% gehemmt wird.

| Verbindung I Beispiel Nr. | IC₅₀ (mg/ml) bei: C. albicans nach: | | C. neoformans 48 Stunden | A. fumigatus 48 Stunden |
|---|---|---|---|---|
| | 24 Std. | 48 Std. | | |
| 1 | < 0,32 | 3.60 | < 0.32 | 3,90 |
| 21 | < 0,32 | 1.30 | < 0.32 | 4,10 |
| 24 | < 0,32 | 1,40 | <0,32 | 1.90 |
| 29a | < 0,32 | 1,10 | < 0,32 | 0,37 |
| 34 | 1,6 | 6,50 | <0,32 | 3,90 |

Die Verbindungen der Formel I und ihre pharmazeutisch annehmbaren Säureadditionssalze können als Heilmittel, z.B. in Form pharmazeutischer Präparate zur enteralen, parenteralen oder topischen Applikation, Verwendung finden. Sie können beispielsweise peroral, z.B. in Form von Tabletten, Lacktabletten, Dragées, Hart- und Weichgelatinekapseln, Lösungen, Emulsionen oder Suspensionen, rektal, z.B. in Form von Suppositorien, parenteral, z.B. in Form von Injektionslösungen oder Infusionslösungen, oder topisch, z.B. in Form von Salben, Crèmen oder Oelen, verabreicht werden.

Die Herstellung der pharmazeutischen Präparate kann in jedem Fachmann geläufiger Weise dadurch erfolgen, dass man die beschriebenen Verbindungen der Formel I und ihre pharmazeutisch annehmbaren Säureadditionssalze, gegebenenfalls in Kombination mit anderen therapeutisch wertvollen Stoffen, zusammen mit geeigneten, nicht-toxischen, inerten, therapeutisch verträglichen festen oder flüssigen Trägermaterialien und gegebenenfalls den üblichen pharmazeutischen Hilfsstoffen in eine galenische Darreichungsform bringt.

Als Trägermaterialien eignen sich sowohl anorganische als auch organische Trägermaterialien. So kann man für Tabletten, Lacktabletten, Dragées und Hartgelatinekapseln beispielsweise Lactose, Maisstärke oder Derivate davon, Talk, Stearinsäure oder deren Salze als Trägermaterialien verwenden. Für Weichgelatinekapseln eignen sich als Trägermaterialien beispielsweise pflanzliche Oele, Wachse, Fette und halbfeste und flüssige Polyole (je nach Beschaffenheit des Wirkstoffes sind jedoch bei Weichgelatinekapseln keine Träger erforderlich). Zur Herstellung von Lösungen und Sirupen eignen sich als Trägermaterialien beispielsweise Wasser, Polyole, Saccharose, Invertzucker und Glucose. Für Injektionslösungen eignen sich als Trägermaterialien beispielsweise Wasser, Alkohole, Polyole, Glycerin und pflanzliche Oele. Für Suppositorien eignen sich als Trägermaterialien beispielsweise natürliche oder gehärtete Oele, Wachse, Fette und halbflüssige oder flüssige Polyole. Für topische Präparate eignen sich als Trägermaterialien Glyceride, halbsynthetische und synthetische Glyceride, hydrierte Oele, flüssige Wachse, flüssige Paraffine, flüssige Fettalkohole, Sterole, Polyäthylenglykole und Cellulosederivate.

Als pharmazeutische Hilfsstoffe kommen die üblichen Stabilisierungs-, Konservierungs-, Netz- und Emulgiermittel, Mittel zur Verbesserung der Konsistenz, Mittel zur geschmacklichen Verbesserung, Salze zur Veränderung des osmotischen Druckes, Puffersubstanzen, Lösungsvermittler, Färbe- und Ueberzugsmittel und Antioxidantien in Frage.

Die Dosierung der Verbindungen der Formel I kann, abhängig von den zu bekämpfenden pathogenen Pilzen, dem Alter und dem individuellen Zustand des Patienten und von der Applikationsweise, innerhalb weiter Grenzen variieren und ist natürlich in jedem einzelnen Fall den individuellen Gegebenheiten anzupassen. Zur Verhütung und Bekämpfung von topischen und systemischen Infektionen durch pathogene Pilze kommt für den erwachsenen Patienten eine tägliche Dosis von etwa 0,01 g bis etwa 4 g, insbesondere etwa 0,05 g bis etwa 2 g in Betracht. Für die Cholesterinsenkung beträgt die Tagesdosis zweckmässigerweise zwischen 1 und 1200 mg, vorzugsweise 5 bis 100 mg, für den erwachsenen Patienten. Je nach Dosierung ist es dabei zweckmässig, die Tagesdosis in mehreren Dosierungseinheiten zu verabreichen.

Die pharmazeutischen Präparate enthalten zweckmässigerweise etwa 1-500 mg, vorzugsweise 2-200 mg einer Verbindung der Formel I.

Die nachfolgenden Beispiele dienen der näheren Erläuterung der vorliegenden Erfindung. Sie sollen deren Umfang jedoch in keiner Weise beschränken. Sämtliche Temperaturen sind in Celsiusgraden angegeben.

### Beispiel A

Aa) 30 ml Nitrobenzol werden in einem Eisbad gekühlt und nacheinander mit 6.6 g Aluminiumchlorid und 10.95 g 4-Brom-2-fluor-benzoylchlorid (hergestellt aus 10 g 4-Brom-2-fluor-benzoesäure und 4.34 ml Oxalylchlorid in Methylenchlorid mit 5 Tropfen DMF als Katalysator) in 20 ml Nitrobenzol bei maximal 6 °C versetzt. Das Gemisch wird gerührt, wonach man 4.5 ml Anisol so zugibt, dass die Temperatur nicht über 6 °C steigt. Die Lösung wird über Nacht auf Raumtemperatur aufwärmen gelassen, auf Eiswasser gegossen und mit Methylenchlorid extrahiert. Die organische Phase wird mit Wasser und 10% Natriumchlorid-Lösung gewaschen, über Natriumsulfat getrocknet und eingeengt. Nach Kristallisation aus Cyclohexan werden 11.3 g (4-Brom-2-fluor-phenyl)-(4-methoxy-phenyl)-methanon mit einem Smp. von 93-94 °C erhalten.
Ab) Eine Lösung von 11.14 g des Produkts von Aa) in 70 ml Essigsäure und 50 ml 62%-iger wässriger HBr-Lösung wird unter Rückfluss gekocht, anschliessend eingedampft, mit Toluol nachgedampft und in Essigester aufgenommen. Die organische Phase wird mit gesättigter Natriumhydrogencarbonat-Lösung und 10% iger Natriumchlorid-Lösung gewaschen und getrocknet. Es werden 10.5 g (4-Brom-2-fluor-phenyl)-(4-hydroxy-phenyl)-methanon erhalten, MS: m/e 294 (M, 1Br).
Ac) 5.25 g des Produkts von Ab) werden in 170 ml Aceton aufgenommen und mit 7.35 g Kaliumcarbonat und 8.13 ml 1,6-Dibromhexan versetzt. Die Suspension wird 4 Std unter Rückfluß erhitzt, dann abgekühlt, filtriert und eingeengt. Nach dem Entfernen des überschüßigen 1,6-Dibromhexans und Kristallisation aus Cyclohexan werden 4.25g [4-[6-Brom-hexyloxy]-phenyl]-(4-brom-2-fluor-phenyl)-methanon erhalten.
Ad) Davon werden 4.1 g in 30 ml Dimethylacetamid (DMA) aufgenommen, bei 0 °C mit 1.77 ml N-Allyl-methyl-amin versetzt, nach einem Tag noch einmal mit 1.77 ml N-Allyl-methyl-amin versetzt und nach zusätzlichen 30 Min eingeengt. Das zurückbleibende Öl wird in Methylenchlorid aufgenommen, mit gesättigter Natriumhydrogencarbonat gewaschen und getrocknet. Das nach dem Reinigen an Kieselgel mit Methylenchlorid: Methanol (95:5) erhaltene [4-[6-(Allyl-methyl-amino)-hexyloxy]-phenyl]-(4-brom-2-fluor-phenyl)-methanon, wird in Äthanol aufgenommen und mit 1.45 g Fumarsäure versetzt. Die homogene Lösung wird eingedampft und getrocknet. Es wird [4-[6-(Allyl-methyl-amino)-hexyloxy]-phenyl]-(4-brom-2-fluor-phenyl)-methanon·Fumarat (1:1) als klebriges Öl erhalten, MS: m/e 447 (M, 1Br).

### Beispiel B

Analog Ac) und Ad) erhält man aus 2-Fluor-4-hydroxyacetophenon, via 1-[4-[6-Brom-hexyloxy]-2-fluor-pheny]-ethanon, das 1-[4-[6-(Allyl-methyl-amino)-hexyloxy]-2-fluor-pheny]-ethanon, welches in das Fumarat überführt wird, MS: m/e 308 (M+H⁺).

### Beispiel 1

### Allyl-[6-[3-(4-brom-phenyl)-benzo[d]isothiazol-6-yloxy]-hexyl]-methyl-amin

a) 4.3 g Kalium- tert. butylat werden in 160 ml THF gelöst und langsam mit 4.5 ml Benzylmercaptan versetzt. Die Suspension wird bei RT gerührt und dann mit 10 g (4-Brom-phenyl)-(2-fluor-4-methoxy-phenyl)-methanon in 200 ml THF versetzt. Die Lösung wird bei RT gerührt, mit 100 ml Ammoniumchloridlösung sowie 200 ml Natriumhydrogencarbonatlösung versetzt. Die Phasen werden getrennt, die anorganische Phase wird mit Essigester extrahiert, und die organischen Phasen werden mit ges. Natriumhydrogencarbonatlösung und Kochsalzlösung gewaschen und getrocknet. Es werden 15.8 g (2-Benzylsulfanyl-4-methoxy-phenyl)-(4-brom-phenyl)-methanon erhalten, die in 160 ml Methylenchlorid aufgenommen werden, mit 3.4 ml Sulfurylchlorid versetzt und bei RT gerührt werden. Nach dem Abdestillieren des Sulfurylchlorid wird der Rückstand in 160 ml THF aufgenommen, mit 120 ml einer gesättigten Ammoniaklösung in Äthanol versetzt und gerührt. Es werden 100 ml Natriumhydrogencarbonatlösung zugegeben. Das Lösungsmittel wird abgezogen und der Rückstand wird erneut in Natriumhydrogencarbonatlösung und Essigester aufgenommen. Die Phasen werden getrennt, und die anorganische Phase wird mit Essigester und Ether extrahiert. Die organischen Phasen werden mit Kochsalzlösung gewaschen und getrocknet. Umkristallisieren aus Essigester/Äthanol liefert 7.52 g 3-(4-Brom-phenyl)-6-methoxy-[d]-benzisothiazol, MS: m/e 318 (M, 1Br).
b) 6.32 g des Produkts von a) werden in 395 ml Methylenchlorid gelöst, auf -78°C abgekühlt und mit 49.3 ml Bortribromid-lösung (1M in Methylenchlorid) versetzt. Die Lösung wird über Nacht aufgetaut und dann bei RT gerührt. Die Lösung wird auf Natriumhydrogencarbonatlösung gegeben, die Phasen werden getrennt und die wäßrige Phase wird mit 150 ml Methylenchlorid extrahiert, die organischen Phasen werden mit Kochsalzlösung gewaschen und getrocknet. Aus dem Rohprodukt werden 1.5 g 3-(4-Brom-phenyl)-benzo[d]isothiazol-6-ol aus Methylenchlorid kristallisert.
   Die Mutterlauge wird eingeengt, in 320 ml Methylenchloird aufgelöst und bei -78°C mit 40 ml Bortribromid-lösung (1 M in Methylenchlorid) behandelt. Nach der Aufarbeitung und der Kristallisation werden weitere 2.24 g 3-(4-Brom-phenyl)-benzo[d]isothiazol-6-ol erhalten.
c) 3.0 g des Produkts von b) werden unter Argon in 50 ml Aceton gelöst und mit 8.73 g Kaliumcarbonat und 3.85 ml 1,6-Dibromhexan versetzt. Die Suspension wird unter Rückfluß erhitzt, dann abgekühlt, mit Methylenchlorid versetzt und abfiltriert. Der Rückstand wird gewaschen. Das Gemisch wird eingeengt, in Methylenchlorid aufgenommen und getrocknet. Das Rohprodukt wid eingeengt und vom überschüssigen 1,6-Dibromhexan befreit. Es werden 4.18 g 6-(6-Brom-hexyloxy)-3-(4-brom-phenyl)-benzo[d]isothiazol als hellbraune Substanz erhalten.
d) 4.15 g des Produkts von c) werden in 40 ml DMA aufgenommen und unter Argon mit 2.6 ml N-Allyl-methyl-amin versetzt und bei RT gerührt. Es werden nochmals 1.7 ml N-Allyl-methyl-amin zugegeben und unter Argon gerührt. Das Lösungsmittel und das überschüssige N-Allyl-methyl-amin werden abgezogen. Das Rohprodukt wird in Methylenchlorid aufgenommen, mit ges. Natriumhydrogencarbonatlösung und Kochsalzlösung gewaschen und getrocknet. Das Rohprodukt wird durch Säulenchromatographie (Kieselgel, Methylenchlorid:Methanol (95:5) gereinigt. Es werden 3.02 g Allyl-[6-[3-(4-brom-phenyl)-benzo[d]isothiazol-6-yloxy]-hexyl]-methyl-amin als gelbes Öl erhalten. Dieses wird in 50 ml Äthanol und 7 ml Methylenchlorid gelöst und mit 750 mg Fumarsäure in 20 ml Äthanol versetzt. Die Lösung wird eingeengt, die Substanz erneut in 25 ml Essigester und 3 ml Äthanol aufgenommen, die Lösung auf 60°C erhitzt, dann abgekühlt und kristallisiert. Es werden 3.43 g farbloses Allyl-[6-[3-(4-brom-phenyl)-benzo[d]isothiazol-6-yloxy]-hexyl]-methyl-amin·Fumarat (1:1), Schmelzpunkt von 122-123.5°C erhalten, MS: m/e 459 (M+H⁺, 1Br).

### Beispiel 2

Analog Beispiel 1 erhält man:
a) aus 3-(4-Brom-phenyl)-6-methoxy-[d]-benzisothiazol (Bsp 1a), nach Abspaltung der Methoxyschutzgruppe, Umsetzung mit (E)-1,4-Dibrombuten und N-Allyl-methyl-amin und Salzbildung, via 3-(4-Brom-phenyl)-benzo[d]isothiazol-6-ol und via (E)-6-(4-Brom-but-2-enyloxy)-3-(4-brom-phenyl)-benzo[d]isothiazol das (E)-Allyl-[4-[3-(4-brom-phenyl)-benzo[d]isothiazol-6-yloxy]-but-2-enyl]-methyl-amin·Fumarat (1:1), MS: m/e 429 (M+H⁺, 1Br),
b) aus 3-(4-Brom-phenyl)-6-methoxy-[d]-benzisothiazol (Bsp 1a), nach Abspaltung der Methoxyschutzgruppe, Umsetzung mit 1,6-Dibromhexan und Methyl-(2-methylsulfanyl-ethyl)-amin und Salzbildung, via 3-(4-Brom-phenyl)-benzo[d]isothiazol-6-ol und via 6-(6-Brom-hexyloxy)-3-(4-brom-phenyl)-benzo[d]isothiazol das 6-[3-(4-Brom-phenyl)-benzo[d]isothiazol-6-yloxy]-hexyl]-methyl-(2-methylsulfanyl-ethyl)-amin·Fumarat (1:1), MS: m/e 431 (M-C2H5S⁺, 1Br),
c) aus 3-(4-Brom-phenyl)-6-methoxy-[d]-benzisothiazol (Bsp 1a), nach Abspaltung der Methoxyschutzgruppe, Umsetzung mit (E)-1,4-Dibrombuten und Methyl-(2-methylsulfanyl-ethyl)-amin und Salzbildung, via 3-(4-Brom-phenyl)-benzo[d]isothiazol-6-ol und via (E)-6-(4-Brom-but-2-enyloxy)-3-(4-brom-phenyl)-benzo[d]isothiazol das (E)-[4-[3-(4-Brom-phenyl)-benzo[d]isothiazol-6-yloxy]-but-2-enyl]-methyl-(2-methylsulfanyl-ethyl)-amin·Fumarat (1:1), MS: m/e 401 (M-C2H5S⁺, 1Br),
d) aus 2-Fluor-4-methoxyacetophenon mit Benzylmercaptan, Sulfurylchlorid und Ammoniak in Äthanol, via 1-(2-Benzylsulfanyl-4-methoxy-phenyl)-ethanon das 6-Methoxy-3-methyl-benzo[d]isothiazol: Smp. 75-76°C, MS: m/e 179 (M⁺) und nach Abspaltung der Methoxyschutzgruppe, Umsetzung mit 1,6-Dibomhexan und N-Allyl-methyl-amin und Salzbildung, via 3-Methyl-benzo[d]isothiazol-6-ol und via 6-(6-Brom-hexyloxy)-3-methyl-benzo[d]-isothiazol: das Allyl-methyl-[6-(3-methyl-benzo[d]isothiazol-6-yloxy)-hexyl]-amin·Fumarat (1:1), MS: m/e 318 (M),
e) aus 6-Methoxy-3-methyl-benzo[d]isothiazol (Zwischenprodukt in Bsp 2d), nach Abspaltung der Methoxyschutzgruppe, Umsetzung mit (E)-1,4-Dibrombuten und N-Allyl-methyl-amin und Salzbildung, via 3-Methyl-benzo[d]isothiazol-6-ol und via (E)-6-(4-Brom-but-2-enyloxy)-3-methyl-benzo[d]isothiazol das (E)-Allyl-methyl-[4-(3-methyl-benzo[d]isothiazol-6-yloxy)-but-2-enyl]-amin·Fumarat (1:1), MS: m/e 289 (M+H⁺),
f) aus (4-Brom-2-fluor-phenyl)-(4-methoxy-phenyl)-methanon (Bsp Aa), via (2-Benzylsufanyl-4-brom-phenyl)-(4-methoxy-phenyl)-methanon und via 6-Brom-3-(4-methoxy-phenyl)-[d]-benzoisothiazol, MS: m/e 319 (M, 1Br), und nach Abspaltung der Methoxyschutzgruppe, Umsetzung mit 1,6-Dibomhexan und N-Allyl-methyl-amin und Salzbildung, via 4-(6-Brom-benzo[d]isothiazol-3-yl)-phenol und via 6-Brom-3-[4-(6-brom-hexyloxy)-phenyl]-benzo[d]isothiazol das Allyl-[6-[4-(6-brom-benzo[d]isothiazol-3-yl)-phenoxy]-hexyl]-methyl-amin·Fumarat (1:1), MS: m/e 458 (M, 1Br),
g) aus 6-Brom-3-(4-methoxy-phenyl)-[d]-benzoisothiazol (Zwischenprodukt in Bsp 2f), nach Abspaltung der Methoxyschutzgruppe, Umsetzung mit (E)-1,4-Dibombuten und N-Allyl-methyl-amin und Salzbildung, via 4-(6-Brom-benzo[d]isothiazol-3-yl)-phenol und via (E)-6-Brom-3-[4-(4-brom-but-2-enyloxy)-phenyl]-benzo[d]isothiazol das (E)-Allyl-[4-[4-(6-brom-benzo[d]-isothiazol-3-yl)-phenoxy]-but-2-enyl]-methyl-amin·Fumarat (1:1), Smp. 125-125.5°C.

### Beispiel 3

### Allyl-[6-[3-(4-brom-phenyl)-benzo[b]thiophen-6-yloxy]-hexyl]-methyl-amin

a) 1.45 g NaSMe (95%) werden in 80 ml THF suspendiert und über einen Zeitraum von 1.5h mit 5.51 g (4-Brom-phenyl)-(2-fluor-4-methoxy-phenyl)-methanon in 100 ml THF versetzt. Die Lösung wird bei RT gerührt, erneut mit 264 mg NaSMe versetzt, weitergerührt und im Abstand von 10 min mit 50 ml ges. NH₄Cl-Lösung und 100 ml Natriumhydrogencarbonatlösung versetzt. Die anorganische Phase wird mit CH₂Cl₂ extrahiert und die organischen Phasen werden mit ges. Natriumhydrogencarbonatlösung sowie Kochsalzlösung gewaschen und getrocknet. Das Rohprodukt wird durch Säulenchromatographie (Kieselgel, Essigester: Hexan 1:2) gereinigt. Es werden 5.88 g (4-Brom-phenyl)-(4-methoxy-2-methylsulfanyl-phenyl)-methanon als gelbes Öl erhalten.
b) 2.55 g Kalium-tert. butylat werden in 15 ml THF vorgelegt und mit 2.50 g des Produkts von a) in 12 ml THF versetzt. Das Gemisch wird unter Rückfluss erhitzt, dann mit Wasser und NH₄Cl versetzt. Die anorganischen Phasen werden mit Essigester extrahiert. Die organischen Phasen werden mit gesättigter Natriumhydrogencarbonatlösung und Kochsalzlösung gewaschen und getrocknet. Das nach dem Einengen erhaltene Rohprodukt (2.08 g) wird in 14 ml Toluol gelöst und mit 6 ml Trifluoressigsäure bei 0°C versetzt. Das Gemisch wird mit gesättigter Natriumhydrogencarbonatlösung neutralisiert. Die anorganische Phase wird mit Essigester extrahiert. Die organischen Phasen werden mit ges. Natriumhydrogencarbonatlösung und Kochsalzlösung gewaschen und getrocknet. Das erhaltene Rohprodukt wird durch Säulenchromatographie (Kieselgel, Essigester:Hexan 1:6) gereinigt und aus Essigester:Hexan kristallisiert. Es werden 476 mg 3-(4-Brom-phenyl)-6-methoxy-benzo[b]thiophen als weiße Kristalle, Smp. 102.5-104.5°C, MS: m/e 318 (M, Br) erhalten.
c) Aus 3-(4-Brom-phenyl)-6-methoxy-benzo[b]thiophen werden nach Abspaltung der Methoxyschutzgruppe (analog Bsp Ab) durch Umsetzung mit 1,6-Dibromhexan (analog Bsp Ac) und N-Allyl-methyl-amin und Salzbildung (analog Bsp Ad) via 3-(4-Brom-phenyl)-benzo[b]thiophen-6-ol und via 6-(6-Brom-hexyloxy)-3-(4-brom-phenyl)-benzo[b]thiophen, das Allyl-[6-[3-(4-brom-phenyl)-benzo[b]thiophen-6-yloxy]-hexyl]-methyl-amin·Fumarat (1:1), MS: m/e 457 (M+H⁺, Br) erhalten.

### Beispiel 4

Analog Beispiel 3 erhält man aus 3-(4-Brom-phenyl)-6-methoxy-benzo[b]thiophen (Bsp 3b) nach Abspaltung der Methoxyschutzgruppe, Umsetzung mit (E)-1,4-Dibrombuten und N-Allyl-methyl-amin und Salzbildung, via 3-(4-Brom-phenyl)-benzo[b]thiophen-6-ol und via 6-(4-(E)-Brom-but-2-enyloxy)-3-(4-brom-phenyl)-benzo[b]thiophen, das (E)-Allyl-[4-[3-(4-brom-phenyl)-benzo[b]thiopen-6-yloxy]-but-2-enyl]-methyl-amin· Fumarat (1:1), MS: m/e 428 (M+H⁺, Br).

### Beispiel 5

### Allyl-methyl-[6-[3-(4-methyl-pent-3-enyl)-benzo[d]isothiazol-6-yloxy]-hexyl-amin

105 mg Allyl-methyl-[6-(3-methyl-benzo[d]isothiazol-6-yloxy)-hexyl]-amin (Bsp 2d) in 2 ml THF werden über einen Zeitraum von 20 min bei -78°C zu einer zuvor aus 100 µl Diisopropylamin und 425 µl n-Butyllithium (1.6 M in Hexan) in 2 ml THF hergestellten Lithiumdiisopropylamid (LDA)-Lösung getropft. Das Gemisch wird bei -78°C gerührt, mit 3,3-Dimethylallylbromid versetzt und über Nacht aufgetaut. Zur Aufarbeitung wird erneut auf -78°C abgekühlt und mit 42 µl Essigsäure in 0.3 ml Ether versetzt. Das Gemisch wird auf eiskalte Natriumhydrogencarbonatlösung gegeben, die anorganische Phase wird mit Methylenchlorid extrahiert. Die organischen Phasen werden mit Kochsalzlösung gewaschen und getrocknet. Das nach dem Einengen erhaltene Rohprodukt wird durch Säulenchromatographie (Kieselgel, CH₂Cl₂:MeOH:NH₄OH 95:5:0.5) gereinigt. Es werden 63 mg Allyl-methyl-[6-[3-(4-methyl-pent-3-enyl)-benzo[d]isothiazol-6-yloxy]-hexyl-amin erhalten. Dieses wird in 2 ml Äthanol aufgenommen und mit 18.1 mg Fumarsäure versetzt. Nach Rühren wird das Lösungsmittel abgezogen und der Rückstand lyophilisiert. Es werden 80 mg Allyl-methyl-[6-[3-(4-methyl-pent-3-enyl)-benzo[d]isothiazol-6-yloxy]-hexyl-amin·Fumarat (1:1) erhalten, MS: m/e 357 (M+H⁺).

### Beispiel 6

Analog Beipiel 5 erhält man aus (E)-Allyl-methyl-[4-(3-methyl-benzo[d]isothiazol-6-yloxy)-but-2-enyl]-amin (Bsp 2e) und 3,3-Dimethyl-allylbromid mit LDA in THF und anschließender Salzbildung das (E)-Allyl-methyl-[4-[3-(4-methyl-pent-3-enyl)-benzo[d]isothiazol-6-yloxy]-but-2-enyl]-amin·Fumarat (1:1), MS: m/e 357 (M+H⁺).

### Beispiel 7

### (E)-Allyl-methyl-[6-[3-(4-methyl-penta-1,3-dienyl)-benzo[d]isothiazol-6-yloxy]-hexyl]-amin

a) 7.0 g 6-Methoxy-3-methyl-benzo[d]isothiazol (Zwischenprodukt in Bsp 2d) werden in 77 ml Essigsäure gelöst und mit 45 ml 62% wässriger HBr versetzt und über Nacht auf 125°C erhitzt. Die Lösung wird mit Natriumhydrogencarbonatlösung versetzt. Die anorganische Phase wird mit Essigester extrahiert, die organischen Phasen werden mit Natriumhydrogencarbonatlösung und Kochsalzlösung gewaschen und getrocknet. Aus CH₂Cl₂ werden 5.12g 3-Methyl-benzo[d]isothiazol-6-ol kristallisiert.
b) 1.5 g 3-Methyl-benzo[d]isothiazol-6-ol in 26 ml THF werden zu einer zuvor aus 3.9 ml Diisopropylamin und 16.0 ml n-Butyllithium (1.6 M in Hexan) in 23 ml THF dargestellten LDA-lösung gegeben und bei -78°C gerührt. 2.5 ml 3-Methyl-2-butenal in 40 ml THF werden zugegeben. Das Reaktionsgemisch wird über Nacht aufgetaut und mit 3.9 ml Essigsäure in 20 ml Ether neutralisiert. Das Gemisch wird auf Natriumhydrogencarbonatlösung gegeben, die anorganische Phase wird mit Essigester extrahiert und die organischen Phasen werden mit Kochsalzlösung gewaschen und getrocknet. Das Rohprodukt wird durch Chromatographie (Kieselgel, Essigester:Hexan 1:1) gereinigt. Es werden 1.5 g (RS)-3-(2-Hydroxy-4-methyl-pent-3-enyl)-benzo[d]isothiazol-6-ol erhalten.
c) 308 mg des Produkts von b) in einem 1:1 Gemisch aus Toluol und THF werden mit 285 mg p-Toluolsulfonsäure in 7 ml Toluol behandelt. Nach 48 h wird weitere p-Toluolsulfonsäure zugesetzt und die Reaktion 6h auf 60°C erhitzt. Nach der Zugabe von Wasser wird die anorganische Phase mit Essigester extrahiert. Die organischen Phasen werden mit Natriumhydrogencarbonatlösung und Kochsalzlösung gewaschen und getrocknet. Es werden 296 mg eines Gemisches von 3-(4-Methyl-penta-2,4-dienyl)-benzo[d]isothiazol-6-ol und 3-(4-Methyl-penta-1,3-dienyl)-benzo[d]isothiazol-6-ol erhalten, MS: m/e 357 (M+H⁺).
d) 307 mg des Produkts von c) werden in 12 ml Toluol gelöst, mit 1 ml Trifluoressigsäure versetzt und bei 60°C gerührt. Die Reaktionslösung wird auf Natriumcarbonatlösung gegossen, die anorganische Phase wird mit Essigester extrahiert, und die organischen Phasen werden mit Natriumhydrogencarbonatlösung und Kochsalzlösung gewaschen und getrocknet. Das Rohprodukt wird durch Chromatographie (Kieselgel, Essigester:Hexan 1:2) gereinigt. Es werden 190 mg 3-(4-Methyl-penta-1,3-dienyl)-benzo[d]isothiazol-6-ol erhalten.
e) Aus dem Produkt von c) erhält man mit 1,6-Dibromhexan (analog Bsp 1c) und N-Allyl-methyl-amin und Salzbildung (analog Bsp 1d), via 6-(6-Brom-hexyloxy)-3-(4-methyl-penta-1,3-dienyl)-benzo[d]isothiazol, das (E)-Allyl-methyl-[6-[3-(4-methyl-penta-1,3-dienyl)-benzo[d]isothiazol-6-yloxy]-hexyl]-amin·Fumarat (1:1), MS: m/e 385 (M+H⁺).

### Beispiel 8

Analog Beispiel 7 erhält man:
a) aus (RS)-3-(2-Hydroxy-4-methyl-pent-3-enyl)-benzo[d]isothiazol-6-ol (Bsp 7b), mit 1,6-Dibromhexan und N-Allyl-methyl-amin und Salzbildung (analog Bsp 1d) via (RS)-1-[6-(6-Brom-hexyloxy)-benzo[d]isothiazol-3-yl]-4-methyl-pent-3-en-2-ol: das (RS)-1-[6-[6-(Allyl-methyl-amino)-hexyloxy]-benzo[d]-isothiazol-3-yl]-4-methyl-pent-3-en-2-ol·Fumarat (1:1), MS: m/e 403 (M+H⁺),
b) aus (RS)-3-(2-Hydroxy-4-methyl-pent-3-enyl)-benzo[d]isothiazol-6-ol (Bsp 7b), mit (E)-1,4-Dibrombuten und N-Allyl-methyl-amin, via (E)-1-[6-(4-Brom-but-2-enyloxy)-benzo[d]isothiazol-3-yl]-4-methyl-pent-3-en-2-ol das (E)-(RS)-1-[6-[4-(Allyl-methyl-amino)-but-2-enyloxy]-benzo[d]isothiazol-3-yl]-4-methyl-pent-3-en-2-ol, MS: m/e 373 (M+H⁺).
c) aus 3-(4-Methyl-penta-2,4-dienyl)-benzo[d]isothiazol-6-ol und 3-(4-Methyl-penta-1,3-dienyl)-benzo[d]isothiazol-6-ol (Bsp 7c), mit (E)-1,4-Dibrombuten und N-Allyl-methyl-amin, via 6-(4-Brom-but-2-enyloxy)-3-(4-methyl-penta-1,3-dienyl)-benzo[d]isothiazol und 6-(4-Brom-but-2-enyloxy)-3-(4-methyl-penta-2,4-dienyl)-benzo[d]isothiazol ein 7:3 Gemisch von Allyl-methyl-[(E)-4-[3-[(E)-4-methyl-penta-2,4-dienyl]benzo[d]isothiazol-6-yloxy]-but-2-enyl]-amin und Allyl-methyl-[(E)-4-[3-[(E)-4-methyl-penta-1,3-dienyl]-benzo[d]isothiazol-6-yloxy]-but-2-enyl]-amin, MS: m/e 355 (M+H⁺).

### Beispiel 9

### 7:3 Gemisch von (E)-Allyl-methyl-[6-[3-(4-methyl-penta-2,4-dienyl)-benzo[d]isothiazol-6-yloxy]-hexyl]-amin und (E)-Allyl-methyl-[6-[3-(4-methyl-penta-1,3-dienyl)-benzo[d]isothiazol-6-yloxy]-hexyl]-amin

Zu 97 mg (RS)-1-[6-[6-(Allyl-methyl-amino)-hexyloxy]-benzo[d]isothiazol-3-yl]-4-methyl-pent-3-en-2-ol (Bsp 8a) in 7 ml Toluol und 5 ml THF werden 116 mg p-Toluolsulfonsäure und nach dem Rühren über Nacht noch 92 mg p-Toluolsulfonsäure zugegeben. Das Reaktionsgemisch wird weitergerührt, dann eingeengt und mit Wasser versetzt. Die anorganische Phase wird mit Methylenchlorid extrahiert. Die organischen Extrakte werden mit NatriumhydrogencarbonatLösung und Kochsalzlösung gewaschen und getrocknet. Das erhaltene Rohprodukt wird durch Säulenchromatographie (Kieselgel, CH₂Cl₂:MeOH) gereinigt. Es werden 75 mg des Titelgemisches erhalten, MS: m/e 385 (M+H⁺).

### Beispiel 10

### Allyl-[6-[4-(6-brom-1,1-dioxo-benzo[d]isothiazol-3-yl)-phenoxy]-hexyl]-methyl-amin

a) 650 mg 6-(6-Brom-hexyloxy)-3-(4-brom-phenyl)-benzo[d]isothiazol (Bsp 1c) werden in 60 ml Methylenchlorid aufgenommen und mit 3 g Kaliumpermanganat (auf Kieselgel) versetzt. Die Suspension wird bei RT gerührt, dann mit Natriumsulfat versetzt und über Kieselgel filtriert. Nach dem Einengen werden aus Essigester, Hexan und Methylenchlorid 439 mg 6-(6-Brom-hexyloxy)-3-(4-brom-phenyl)-benzo[d]isothiazol-1,1-dioxid kristallisiert, MS: m/e 499 (M, 1 Br).
b) Aus dem Produkt von a) und N-Allyl-methyl-amin und nach der Salzbildung erhält man (analog Bsp 1d) das Allyl-[6-[4-(6-brom-1,1-dioxo-benzo[d]isothiazol-3-yl)-phenoxy]-hexyl]-methyl-amin·Fumarat (1:1), MS: m/e 491 (M+H⁺, 1 Br).

### Beispiel 11

Analog Beispiel 10 erhält man aus 6-Brom-3-[4-(6-brom-hexyloxy)-phenyl]-benzo[d]isothiazol (Zwischenprodukt von Bsp 2f) nach Oxidation mit KMnO₄, 6-Brom-3-[4-(6-brom-hexyloxy)-phenyl]-benzo[d]isothiazol-1,1-dioxid, welches mit N-Allyl-methyl-amin und nach Salzbildung (analog Bsp 1d) Allyl-[6-[4-(6-brom-1,1-dioxo-benzo[d]isothiazol-3-yl)-phenoxy]-hexyl]-methyl-amin·Fumarat (1:1), MS: m/e 491 (M+H⁺, 1 Br) ergibt.

### Beispiel 12

a) Ein Gemisch von 4,25g (4-Brom-phenyl)-(2,4-dihydroxy-phenyl)-methanon, 3,22g Hydroxylamin·Hydrochlorid und 2,85g Natriumacetat in 100 ml Äthanol wird unter Rückfluß erhitzt. Das Reaktionsgemisch wird eingeengt, mit 100 ml gesättigter wäßriger Natriumhydrogencarbonatlösung versetzt und mit Essigester extrahiert. Die organischen Auszüge werden mit gesättigter Natriumchloridlösung gewaschen, getrocknet und eingedampft. Der Rückstand wird mit 200 ml Äthanol versetzt. Nach Zugabe von 1,1g p-Toluolsulfonsäure wird 24 Std auf 85°C erwärmt. Zur Aufarbeitung wird das Reaktiongemisch eingeengt und der Rückstand in 250 ml Essigester aufgenommen und mit gesättigter Natriumhydrogencarbonatlösung sowie gesättigter Natriumchloridlösung gewaschen. Die organische Phase wird getrocknet und eingedampft. Es werden 4.0g 3-(4-Brom-phenyl)-benzo[d]isoxazol-6-ol erhalten, MS: m/e 289 (M+H⁺, 1Br).
b) Analog Beispiel 1 erhält man aus 3-(4-Brom-phenyl)-benzo[d]isoxazol-6-ol und (E)-1,4-Dibrom-2-buten, via (E)-6-(4-Brom-but-2-enyloxy)-3-(4-brom-phenyl)-benzo[d]isoxazol und durch Reaktion mit N-Allyl-methyl-amin, das (E)-Allyl-[4-[3-(4-brom-phenyl)-benzo[d]isoxazol-6-yloxy]-but-2-enyl-methyl-amin, welches in das Fumarat überführt wird, MS: m/e 412 (M⁺, 1Br).

### Beispiel 13

Analog Beispiel 12b erhält man aus 3-(4-Brom-phenyl)-benzo[d]isoxazol-6-ol und 1,6-Dibromhexan, via 6-(6-Brom-hexyloxy)-3-(4-brom-phenyl)-benzo[d]isoxazol und durch Reaktion mit N-Allyl-methyl-amin, das Allyl-[6-[3-(4-brom-phenyl)-benzo[d]isoxazol-6-yloxy]-hexyl]-methyl-amin, welches in das Fumarat überführt wird, MS: m/e 443 (M+H⁺, 1Br).

### Beispiel 14

a) Zu 80ml Nitrobenzol werden bei 5°C, 7.2g Aluminiumchlorid gegeben und eine Lösung von 8g 4-Brom-2-chlor-benzoylchlorid in 20ml Nitrobenzol zugetropft, so daß die Temperatur nicht 10°C überschreitet. Nach dem Rühren und der Zugabe von 3.26ml Anisol wird das Reaktionsgemisch auf Raumtemperatur erwärmt, gerührt und anschließend auf Eiswasser gegossen und mit Methylenchlorid extrahiert. Die Extrakte werden mit gesättigter Natriumchloridlösung gewaschen, getrocknet und eingedampft. Es werden 10.0g (4-Brom-2-chlor-phenyl)-(4-methoxy-phenyl)-methanon erhalten, MS: m/e 324 (M+H⁺, 1Br).
b) Eine Mischung von 4.0g des Produkts von a), 3.41g Hydroxylamin· Hydrochlorid und 3.0g Natriumacetat in 80 ml Äthanol wird 3 Tage unter Rückfluß erhitzt. Das Reaktionsgemisch wird eingeengt, mit gesättigter Natriumhydrogencarbonatlösung versetzt und mit Essigester extrahiert. Die organischen Auszüge werden mit gesättigter Natriumchloridlösung gewaschen, getrocknet und eingedampft. Der Rückstand wird in Dimethylacetamid aufgenommen und mit 7.1g Kaliumcarbonat versetzt. Nach dem Rühren bei 120°C wird das Reaktionsgemisch nach dem Abkühlen filtriert. Das Filtrat wird eingeengt und der Rückstand wird durch Chromatographie (Kieselgel, Essigester/Hexan 1:9 bis 2:8) gereinigt. Es werden 3.5g 6-Brom-3-(4-methoxy-phenyl)-benzo[d]isoxazol, MS: m/e 303 (M+H⁺, 1Br) erhalten.
c) Ein Gemisch aus 3.4g des Produkts von b), 80ml Essigsäure und 50ml 62%-riger wässriger Hydrobromidlösung wird 8 Std bei 95°C gerührt, anschließend eingedampft, in Essigester aufgenommen, auf gesättigte Natriumhydrogencarbonatlösung gegossen und mit Essigester extrahiert. Die organischen Phasen werden mit gesättigter Natriumchloridlösung gewaschen, getrocknet und eingedampft. Nach der Chromatographie des Rückstands (Kieselgel, Essigester/Hexan 3:7) erhält man 2.7g 4-(6-Brom-benzo[d]isoxazol-3-yl)-phenol, MS: m/e 289 (M+H⁺, 1Br).
d) Analog Beispiel 1 erhält man aus 4-(6-Brom-benzo[d]isoxazol-3-yl)-phenol und 1,6-Dibromhexan, via 6-Brom-3-[4-(6-brom-hexyloxy)-phenyl]-benzo[d]isoxazol und durch Reaktion mit N-Allyl-methyl-amin, das Allyl-[6-[4-(6-brom-benzo[d]isoxazol-3-yl)-phenoxy]-hexyl-methyl-amin, welches in das Fumarat überführt wird, MS: m/e 442 (M+H⁺, 1Br).

### Beispiel 15

Analog Beispiel 14d erhält man aus 4-(6-Brom-benzo[d]isoxazol-3-yl)-phenol und (E)-1,4-Dibrom-2-buten, via (E)-6-Brom-3-[4-(4-brom-but-2-enyloxy)-phenyl]-benzo[d]isoxazol und durch Reaktion mit N-Allyl-methyl-amin, das (E)-Allyl-[4-[4-(6-brom-benzo[d]isoxazol-3-yl)-phenoxy]-but-2-enyl]-methyl-amin, welches in das Fumarat überführt wird, MS: m/e 413 (M+H⁺, 1Br).

### Beispiel 16

Ein Gemisch von 0.15g (4-Brom-phenyl)-[4-[6-(cyclopropyl-methyl-amino)-hexyloxy]-2-fluor-phenyl]-methanon, 0.17g Hydroxylamin. Hydrochlorid und 0.20g Natriumacetat in 14 ml Äthanol wird 32 Std auf 90°C erwärmt und dann eingeengt. Der Rückstand (enthaltend das dem Ausgangsketon entsprechende Oxim) wird in 50ml Methylenchlorid aufgenommen und mit gesättigter wäßriger Natriumhydrogencarbonatlösung und Natriumchloridlösung gewaschen. Die organische Phase wird getrocknet und eingedampft. Der Rückstand wird in 15ml DMA gelöst und mit 0.22g Kaliumcarbonat versetzt. Das Gemisch wird 5 Std bei 130°C erhitzt. Zur Aufarbeitung wird das Reaktionsgemisch abfiltriert, das Filtrat eingeengt und durch Chromatographie (Kieselgel, Toluol/Aceton/Triethyl-amin 92:7:1) gereinigt. Es werden 0.077g [6-[3-(4-Brom-phenyl)-benzo[d]-isoxazol-6-yloxy]-hexyl]-cyclopropyl-methyl-amin erhalten, die in das Fumarat überführt werden, MS: m/e 443 (M+H⁺, 1Br).

Das Ausgangsmaterial, MS: m/e 447 (M, 1 Br), erhält man durch Reaktion des [4-(6-Brom-hexyloxy)-2-fluor-phenyl]-(4-brom-phenyl)-methanons mit N-Methyl-cyclopropyl-amin·Hydrochlorid.

### Beispiel 17

Analog Beispiel 16 erhält man aus (E)-1-[4-[(E)-4-(Allyl-methyl-amino)-but-2-enyloxy]-2-fluor-phenyl]-5-methyl-4-hexen-1-on und Hydroxylamin· Hydrochlorid und Cyclisierung mit Kaliumcarbonat, das (E)-Allyl-methyl-[4-[3-(4-methyl-pent-3-enyl)-benzo[d]isoxazol-6-yloxy]-but-2-enyl]-amin, das in das Fumarat überführt wird, MS: m/e 341 (M).

Das Ausgangsmaterial erhält man wie folgt:

Eine Lösung von 52.0 ml Diisopropylamin in 600 ml THF wird bei 0 °C mit 230 ml 1.6 M Butyl-Lithium in Hexan tropfenweise versetzt. Nach 1.5 Std bei 0 °C wird auf -78 °C gekühlt und 26.8 g 2-Fluor-4-hydroxyacetophenon in 120 ml THF zugetropft. Nach 1 Std bei -78 °C wird 23.7 ml 3,3-Dimethylallyl-bromid in 24 ml THF zugetropft. Man lässt auf Raumtemperatur aufwärmen, worauf bei -78 °C 34 ml Essigsäure in 100 ml Äther zugespritzt werden. Die Lösung wird auf gesättigte Ammoniumchlorid-Lösung/Äther gegossen und mit 10% iger Natriumchlorid-Lösung gewaschen. Nach Trocknen und Eindampfen der organischen Phase werden aus Äther/Pentan 33.8 g 1-(2-Fluor-4-hydroxy-phenyl)-5-methyl-hex-4-en-1-on, Smp. von 100-101 ° C erhalten. Durch Umsetzung dieser Verbindung mit (E)-1,4-Dibrom-2-buten und dann mit N-Allyl-methyl-amin erhält man das (E)-1-[4-[(E)-4-(Allyl-methyl-amino)-but-2-enyloxy]-2-fluor-phenyl]-5-methyl-4-hexen-1-on, MS: m/e 345 (M).

### Beispiel 18

a) 11.5g 4-Bromphenacylbromid werden in 250ml Aceton gelöst und nach der Zugabe von 5.75g Kaliumcarbonat und 4.5ml 3-Methoxyphenol wird die Reaktionsmischung gerührt und anschließend abfiltriert. Das Filtrat wird eingeengt und der Rückstand wird über Kieselgel mit Essigester/Hexan (5:95 bis 10:90) chromatographiert. Es werden 7.3g 1-(4-Brom-phenyl)-2-(3-methoxy-phenoxy)-ethanon erhalten, MS: m/e 320 (M⁺, 1Br).
b) 5.0g des Produkts von a) werden mit 53g Polyphosphorsäure versetzt und auf 80°C erwärmt. Zur Aufarbeitung wird mit gesättigter Natrium-carbonatlösung auf pH∼8 gestellt, dann abgekühlt, anschließend mit Essigester extrahiert. Die organischen Auszüge werden getrocknet und eingeengt. Es werden 4.6g 3-(4-Brom-phenyl)-6-methoxy-benzofuran erhalten, MS: m/e 302 (M⁺, 1Br).
c) 3.0g 3-(4-Brom-phenyl)-6-methoxy-benzofuran werden bei 0°C mit 100ml 1M Bromtribromidlösung in Methylenchlorid versetzt. Das Eisbad wird entfernt und das Reaktiongemisch gerührt, anschließend auf gesättigte Natriumcarbonatlösung gegossen und mit Methylenchlorid extrahiert. Die organischen Phasen werden mit gesättigter Natriumchloridlösung gewaschen, getrocknet und eingedampft. Es werden 2.9g 3-(4-Brom-phenyl)-benzofuran-6-ol erhalten, MS: m/e 288 (M⁺, 1Br).
d) Analog Beispiel 1 erhält man aus 3-(4-Brom-phenyl)-benzofuran-6-ol und (E)-1,4-Dibrom-2-buten, via (E)-6-(4-Brom-but-2-enyloxy)-3-(4-brom-phenyl)-benzofuran und durch Reaktion mit N-Allyl-methyl-amin, das (E)-Allyl-[4-[3-(4-brom-phenyl)-benzofuran-6-yloxy]-but-2-enyl]-methyl-amin, das in das Fumarat überführt wird, MS: m/e 412 (M+H⁺, 1Br).

### Beispiel 19

Analog Beispiel 18d erhält man aus 3-(4-Brom-phenyl)-benzofuran-6-ol und 1,6-Dibromhexan, via 6-(6-Brom-hexyloxy)-3-(4-brom-phenyl)-benzofuran und durch Reaktion mit N-Allyl-methyl-amin, das Allyl-[6-[3-(4-brom-phenyl)-benzofuran-6-yloxy]-hexyl]-methyl-amin, das in das Fumarat überführt wird, MS: m/e 442 (M+H⁺, 1Br).

### Beispiel 20

a) Zu einer Lösung von 43.9g 4-Brombenzoylchlorid in 500ml Methylen-chlorid werden bei 0°C nacheinander 29.2ml 2-(4-Methoxyphenyl)-äthylamin und 34.2ml Diisopropyläthylamin zugetropft. Das Reaktionsgemisch wird auf Raumtemperatur aufwärmen gelassen und gerührt, anschließend mit Methylenchlorid verdünnt und mit gesättigter Natriumhydrogencarbonatlösung versetzt. Die organische Phase wird mit gesättigter Natriumhydrogencarbonatlösung und dann gesättigter Natriumchloridlösung gewaschen, getrocknet und eingeengt. Es werden 66,1g 4-Brom-N-[2-(3-methoxy-phenyl)-ethyl]-benzamid erhalten, MS: m/e 333 (M⁺, 1Br).
b) 50g 4-Brom-N-[2-(3-methoxy-phenyl)-ethyl]-benzamid werden in 750ml Acetonitril gelöst und unter Argon mit 55ml Phosphoroxychlorid versetzt. Nach Erwärmen auf 80°C wird der größte Teil des Lösungsmittel abgezogen, mit konzentrierter Ammoniak-Lösung auf pH >12 gestellt und mit Essigester extrahiert. Die organischen Auszüge werden mit gesättigter Natriumchloridlösung gewaschen, getrocknet und eingeengt. Es werden 46.4g 1-(4-Brom-phenyl)-6-methoxy-3,4-dihydro-isoquinolin erhalten, MS: m/e 314 (M⁺, 1Br).
c) Die Abspaltung der Methoxyschutzgruppe (analog Bsp 14c) des 1-(4-Brom-phenyl)-6-methoxy-3,4-dihydro-isoquinolin (15g) erfolgt mit 95ml Essigsäure und 65ml 62% wässriger Hydrobromid-Lösung bei 100°C und ergibt 13.7g 1-(4-Brom-phenyl)-3,4-dihydro-isoquinolin-6-ol, MS: m/e 301 (M⁺, 1Br).
d) Analog Beispiel 1 erhält man aus 1-(4-Brom-phenyl)-3,4-dihydro-isoquinolin-6-ol und 1,6-Dibromhexan das 6-(6-Brom-hexyloxy)-1-(4-brom-phenyl)-3,4-dihydro-isoquinolin und nach Reaktion mit N-Allyl-methyl-amin das Allyl-[6-[1-(4-brom-phenyl)-3,4-dihydro-isoquinolin-6-yloxy]-hexyl]-methyl-amin, das in das Fumarat überführt wird, MS: m/e 455 (M+H⁺, 1Br).

### Beispiel 21

0.3g Allyl-[6-[1-(4-brom-phenyl)-3,4-dihydro-isoquinolin-6-yloxy]-hexyl]-methyl-amin (Bsp 20d) werden in 50ml Äthanol gelöst und mittels Eisbad auf 0°C gekühlt. In drei Portionen werden innerhalb von 10 Minuten insgesamt 0.185g Natriumborhydrid zugegeben, es wird auf Raumtemperatur erwärmt und über Nacht gerührt. Zur Aufarbeitung wird das Reaktionsgemisch auf 1/4 eingedampft und mit 2 M Salzsäure-Lösung auf pH∼2 gestellt, wieder mit konzentriert Ammoniak-Lösung basisch gestellt und dreimal mit Methylenchlorid extrahriert. Nach dem Eindampfen der organichen Extrakte und Chromatographie des Rückstandes an Kieselgel mit Toluol/Aceton/Triethylamin (50:49:1) als Eluens werden 0.23g (RS)-Allyl-[6-[1-(4-brom-phenyl)-1,2,3,4-tetrahydro-isoquinolin-6-yloxy]-hexyl]-methyl-amin erhalten, die in das Hydrochlorid überführt werden, MS: m/e 457 (M+H⁺, 1Br).

### Beispiel 22

0.1g (RS)-Allyl-[6-[1-(4-brom-phenyl)-1,2,3,4-tetrahydro-isoquinolin-6-yloxy]-hexyl]-methyl-amin (Bsp 21) werden in 2ml einer 1M NaH₂PO₃-Lösung aufgenommen und mit 2ml Dioxan als Lösungsvermittler versetzt. Nach Zugabe von 2ml einer 37% wäßrigen Formaldehydlösung wird 48 Std auf 65°C erwärmt. Zur Aufarbeitung wird mit 4M wäßriger Natronlauge auf pH>12 gestellt und mit Essigester extrahiert. Nach dem Eindampfen der Extrakte und Chromatographie des Rückstandes (Kieselgel, Toluol/Aceton/Triethylamin, 25:74:1) werden 0.12g (RS)-Allyl-[6-[1-(4-brom-phenyl)-2-methyl-1,2,3,4-tetrahydro-isoquinolin-6-yloxy]-hexyl]-methyl-amin erhalten, die ins Hydrochlorid überführt werden, MS: m/e 471 (M+H⁺, 1Br).

### Beispiel 23

a) 10g 1-(4-Brom-phenyl)-6-methoxy-3,4-dihydro-isoquinolin (Bsp 20b) werden in 500ml Toluol gelöst, mit 60g wasserfreiem Natriumsulfat und 15g Mangandioxid versetzt und 24 Std unter Rückfluß gekocht. Nach dem Abkühlen wird das Reaktionsgemisch filtriert und eingeengt.
b) Das erhaltene 1-(4-Brom-phenyl)-6-methoxy-isoquinolin wird in 53ml Essigsäure und 37ml 62%-iger wässriger Bromwasserstoffsäure unter Rückfluß gekocht. Anschließend wird nach Abkühlen auf Raumtemperatur und Zugabe von 150ml Essigester von ausfallendem Niederschlag abfiltriert. Die Kristalle werden mit Essigester gewaschen und getrocknet. Es werden 7.6g 1-(4-Brom-phenyl)-isoquinolin-6-ol erhalten, MS: m/e 300 (M+H⁺, 1Br).
c) Analog Beispiel 1 wird aus 1-(4-Brom-phenyl)-isoquinolin-6-ol und 1,6-Dibromhexan das 6-(6-Brom-hexyloxy)-1-(4-brom-phenyl)-isoquinolin und nach Reaktion mit N-Allyl-methyl-amin das Allyl-[6-[1-(4-brom-phenyl)-isoquinolin-6-yloxy]-hexyl]-methyl-amin erhalten, welches in das Fumarat und das Dihydrochlorid überführt wird, MS: m/e 453 (M+H⁺, 1Br).

### Beispiel 24

Analog Beispiel 23c erhält man aus 1-(4-Brom-phenyl)-isoquinolin-6-ol und 1,6-Dibromhexan das 6-(6-Brom-hexyloxy)-1-(4-brom-phenyl)-isoquinolin und nach Reaktion mit N-Cyclopropyl-methyl-amin das [6-[1-(4-Brom-phenyl)-isoquinolin-6-yloxy]-hexyl]-cyclopropyl-methyl-amin, das in das Fumarat überführt wird, MS: m/e 453 (M+H⁺, 1Br).

### Beispiel 25

Eine Suspension von 0.5g 1-(4-Brom-phenyl)-isoquinolin-6-ol in 15ml DMF wird auf 0°C abgekühlt und mit 0.15g ∼55% Natriumhydrid versetzt. Das Gemisch wird 40 Min gerührt, wobei eine Lösung entsteht. Bei -20°C wird eine Lösung von 0.39g (E)-1,4-Dibrom-2-buten in 5ml DMF zugetropft. Nach 3 Std Rühren bei -15 bis -20°C wird 0.8ml N-Allyl-methyl-amin zugegeben, über Nacht weitergerührt und auf Raumtemperatur erwärmt. Anschließend wird das Reaktionsgemisch eingeengt und der Rückstand mit Natriumhydrogencarbonatlösung versetzt und mit Methylenchlorid extrahiert. Die organischen Extrakte werden mit gesättigter Natriumchloridlösung gewaschen, über Natriumsulfat getrocknet und eingeengt. Der Rückstand wird mit Toluol/Aceton/Triäthylamin (90:9:1 bis 70:29:1) chromatographiert. Es werden 0.1g (E)-Allyl-[4-[1-(4-brom-phenyl)-isoquinolin-6-yloxy]-but-2-enyl]-methyl-amin erhalten, die in das Fumarat überführt werden, MS: m/e 423 (M+H⁺, 1Br).

### Beispiel 26

Analog Beispiel 25 erhält man aus 1-(4-Brom-phenyl)-3,4-dihydro-isoquinolin-6-ol und (E)-1,4-Dibrom-2-buten und nach Reaktion mit N-Allyl-methyl-amin das (E)-Allyl-[4-[1-(4-brom-phenyl)-3,4-dihydro-isoquinolin-6-yloxy]-but-2-enyl]-methyl-amin, welches in das Fumarat überführt wird, MS: m/e 425 (M+H⁺, 1Br).

### Beispiel 27

a) Eine Lösung von 3g (4-Brom-phenyl)-(2-methyl-4-methoxy-phenyl)-methanon (das aus 3-Methylanisol und 4-Brombenzoylchlorid durch Friedel-Crafts Reaktion analog Bsp 14a hergestellt wird) in 100ml Tetrachlormethan wird mit 2.1g N-Bromsuccinimid und einer Spatelspitze Dibenzoylperoxid versetzt. Das Reaktionsgemisch wird unter Bestrahlung bei Raumtemperatur 6 Std gerührt, anschließend mit 100ml Methylenchlorid verdünnt und nacheinander mit gesättigter Natriumhydrogencarbonatlösung und gesättigter Natriumchloridlösung gewaschen. Die organische Phase wird getrocknet, abfiltriert und sofort umgesetzt. Die Lösung wird zu einem zuvor bei 0°C gerührten Gemisch aus 2.13g N-Hydroxy-carbamidsäure-tert-butylester und 0.39g 55% Natriumhydrid in 30ml DMF getropft. Das Reaktionsgemisch wird auf Raumtemperatur erwärmt und gerührt. Zur Aufarbeitung wird mit 60ml gesättigter Ammoniumchloridlösung versetzt und die abgetrennte organische Phase mit gesättigter Natriumchloridlösung gewaschen, über Natriumsulfat getrocknet und eingeengt. Nach Chromatographie des Rückstandes (Kieselgel, Essigester-Hexan, 10:90 bis 25:75) werden 2,65g [2-(4-Brom-benzoyl)-5-methoxy-benzyloxy]-carbamidsäure tert-butylester erhalten, MS: m/e 436 (M+H⁺, 1Br).
b) Eine Lösung von 1.9g des Produkts von a) in 57ml Essigsäure wird mit 38ml 62%-iger wässriger Bromwasserstoffsäure versetzt. Das Gemisch wird für insgesamt 3.5 Tage unter Rückfluß gekocht, anschließend eingeengt und der Rückstand in Essigester aufgenommen, mit Natriumhydrogencarbonatlösung und gesättigter Natriumchloridlösung gewaschen. Die organiche Phase wird getrocknet und eingeengt. Es werden 1.2g 4-(4-Brom-phenyl)-1H-benzo[d][1,2]oxazin-7-ol erhalten, MS: m/e 302 (M-H⁻, 1Br).
c) Ein Gemisch aus 1.28g 4-(4-Brom-phenyl)-1H-benzo[d][1,2]oxazin-7-ol, 2.71g (E)-1,4-Dibrom-2-buten und 1.74g Kaliumcarbonat in 80ml Aceton wird bei 65°C gerührt. Nach dem Abkühlen wird das Reaktionsgemisch abfiltriert, das Filtrat eingeengt und durch Chromatographie (Kieselgel, Toluol/Aceton 98:2) gereinigt. Es werden 1.44g 7-(4-Brom-but-2-enyloxy)-4-(4-brom-phenyl)-1H-benzo[d][1,2]oxazin erhalten MS: m/e 437 (64%, M⁺, 2Br).
d) 1.35g 7-(4-Brom-but-2-enyloxy)-4-(4-brom-phenyl)-1H-benzo[d][1,2]oxazin werden in 50ml Aceton gelöst und mit 3.83ml N-Allyl-methyl-amin und 1.28g Kaliumcarbonat versetzt. Das Gemisch wird gerührt und anschließend abfiltriert. Nach Einengen, des Filtrats wird über Kieselgel mit Toluol/Aceton/Triäthylamin (92:7:1) chromatographiert. Es werden 1.09g (E)-Allyl-[4-[4-(4-brom-phenyl)-1H-benzo[d][1,2]oxazin-7-yloxy]-but-2-enyl]-methyl-amin erhalten, die in das Fumarat (Ro-61-1979/001) überführt werden, MS: m/e 427 (M+H⁺, 1Br).

### Beispiel 28

Analog Beispiel 27 erhält man:
a) aus 4-(4-Brom-phenyl)-1H-benzo[d][1,2] oxazin-7-ol und 1,6-Dibromhexan das 7-(6-Brom-hexyloxy)-4-(4-brom-phenyl)-1H-benzo[d][1,2] oxazin und durch Reaktion mit N-Allyl-methyl-amin: das Allyl-6-[4-(4-brom-phenyl)-1H-benzo[d][1,2]oxazin-7-yloxy]-hexyl-methyl-amin, das in das Fumarat überführt wird, MS: m/e 457 (M+H⁺, 1Br),
b) aus Anisol und 4-Brom-2-methyl-benzoylchlorid das (4-Brom-2-methyl-phenyl)-(4-methoxy-phenyl)-methanon, das nach Bromierung, Addition des N-Hydroxy-carbamidsäure-tert-butylesters, anschließender Cyclisierung und Abspaltung der Methoxyschutzgruppe: das 4-(7-Brom-1H-benzo[d][1,2]oxazin-4-yl)-phenols ergibt, MS: m/e 303 (M⁺, 1Br), das mit 1,6-Dibromhexan das 7-Brom-4-[4-(6-brom-hexyloxy)-phenyl]-1H-benzo[d][1,2]-oxazin und durch Reaktion mit N-Allyl-methyl-amin zu dem Allyl-[6-[4-(7-brom-1H-benzo[d][1,2]oxazin-4-yl)-phenoxy]-hexyl]-methyl-amin umgesetzt wird, welches in das Fumarat überführt wird, MS: m/e 457 (M+H⁺, 1Br),
c) aus 4-(7-Brom-1H-benzo[d][1,2]oxazin-4-yl)-phenol (Zwischenprodukt in Bsp 28b) und (E)-1,4-Dibrombuten das 7-Brom-4-[4-(4-brom-but-2-enyloxy)-phenyl]-1H-benzo [d][1,2] oxazin und durch Reaktion mit N-Allyl-methyl-amin: das (E)-Allyl-[4-[4-(7-brom-1H-benzo[d][1,2]oxazin-4-yl)-phenoxy]-but-2-enyl-methyl-amin, welches in das Fumarat überführt wird, MS: m/e 427 (M+H⁺, 1Br)

### Beispiel 29

a) Eine Lösung von 3g (4-Brom-phenyl)-(2-methyl-4-methoxy-phenyl)-methanon (Zwischenprodukt in Bsp 27a) in 250ml Tetrachlormethan wird mit 3,9g N-Bromsuccinimid und einer Spatelspitze Dibenzoylperoxid versetzt. Das Reaktionsgemisch wird bestrahlt, bei Raumtemperatur gerührt und anschließend von ausgefallenem Niederschlag abfiltriert. Aus dem Filtrat werden nach Waschen mit gesättigter Natriumhydrogencarbonatlösung und Natriumchloridlösung und Einengen 4.5g (4-Brom-phenyl)-(2-dibromomethyl-4-methoxy-phenyl)-methanon isoliert, MS: m/e 460 (M⁺, 2Br).
b) Die Abspaltung der Methoxyschutzgruppe erfolgt analog Bsp. 1b mit 50ml 1M BBr₃ in Methylenchlorid und ergibt 4.0g (4-Brom-phenyl)-(2-dibromo-methyl-4-hydroxy-phenyl)-methanon, MS: m/e 446 (M⁺, 2Br).
c) Diese Verbindung wird mit 200ml 85% Ameisensäure versetzt und 1.5 Std auf 90°C erwärmt. Nach Einengen wird der Rückstand in Methylenchlorid aufgenommen und mit gesättigter Natriumhydrogencarbonatlösung sowie Natriumchloridlösung gewaschen. Die organische Phase wird getrocknet und eingeengt. Es werden 2.5g 2-(4-Brom-benzoyl)-5-hydroxy-benzaldehyd erhalten, MS: m/e 304 (M⁺, 1Br).
d) Analog Beispiel 27c erhält man aus 2-(4-Brom-benzoyl)-5-hydroxy-benzaldehyd und 1,6-Dibromhexan das 2-(4-Brom-benzoyl)-5-(6-brom-hexyloxy)-benzaldehyd und durch Reaktion mit N-Allyl-methyl-amin den 5-[6-(Allyl-methyl-amino)-hexyloxy]-2-(4-brom-benzoyl)-benzaldehyd, MS: m/e 458 (M+H⁺, 1Br).
e) 0,22g des Aldehyds von d) werden mit 0.12ml Hydrazinhydrat in 10ml Äthanol bei Raumtemperatur gerührt. Anschließend wird das Reaktionsgemich eingeengt und der Rückstand in Methylenchlorid aufgenommen, mit gesättigter Natriumhydrogecarbonatlösung und Natriumchloridlösung gewaschen, über Natriumsulfat getrocknet und eingeengt. Es werden 0.2g Allyl-[6-[1-(4-brom-phenyl)-phthalazin-6-yloxy]-hexyl]-methyl-amin erhalten, die ins Fumarat überführt werden, MS: m/e 454 (M+H⁺, 1Br).

### Beispiel 30

Analog Beispiel 29 erhält man aus 2-(4-Brom-benzoyl)-5-hydroxy-benzaldehyd und (E)-1,4-Dibrombuten das 2-(4-Brom-benzoyl)-5-(4-brom-but-2-enyloxy)-benzaldehyd und dann durch Reaktion mit N-Allyl-methyl-amin den 5-[4-(Allyl-methyl-amino)-but-2-enyloxy]-2-(4-brom-benzoyl)-benzaldehyd MS: m/e 428 (M+H⁺, 1Br).

Letzterer ergibt mit Hydrazinhydrat das (E)-Allyl-[4-[1-(4-brom-phenyl)-phthalazin-6-yloxy]-but-2-enyl]-methyl-amin, welches ins Fumarat und ins Hydrochlorid überführt wird, MS: m/e 424 (M+H⁺, 1Br).

### Beispiel 31

### (E)-Allyl-[4-[3-(4-brom-phenyl)-5-fluor-1-methyl-1H-indazol-6-yloxy]-but-2-enyl]-methyl-amin

Eine Mischung von 0.53 ml Methylhydrazin und 0.17 g Kaliumcarbonat wird in 5 ml DMA bei Raumtemperatur gerührt. Nach Zugabe von 0.44 g (E)-[4-[4-(Allyl-methyl-amino)-but-2-enyloxy]-2,5-difluor-phenyl]-(4-brom-phenyl)-methanon in 5 ml DMA wird bei 120 °C gekocht. Nach der Filtration wird bei 80 °C/0.3 Torr eingeengt, in Methylenchlorid aufgenommen, über Natriumsulfat filtriert und eingeengt. Der Rückstand bestehend aus der Titelverbindung wird in Äther/Hexan gelöst, mit 0.11 g Fumarsäure versetzt und gerührt. Nach dem Abfiltrieren und Trocknen erhält man 0.41 g (E)-Allyl-[4-[3-(4-brom-phenyl)-5-fluor-1-methyl-1H-indazol-6-yloxy]-but-2-enyl]-methyl-amin·Fumarat, Smp. 135-137 °C.

0.21 g des Fumarats werden mit Methylenchlorid/gesättigter Natriumbicarbonat-Lösung in die freie Base übergeführt und in Methylenchlorid bei 0°C mit 4.8M Salzsäure in Äther versetzt. Nach Eindampfen werden 0.15 g (E)-Allyl-[4-[3-(4-brom-phenyl)-5-fluor-1-methyl-1H-indazol-6-yloxy]-but-2-enyl]-methyl-amin·Hydrochlorid erhalten, MS: m/e 444 (M+H⁺, 1Br).

Das Ausgangsmaterial (Smp. des Hydrochlorids: 150°C) erhält man aus 4-Brombenzoylchlorid und 2,5-Difluoranisol und (E)-1,4-Dibrombuten, via (4-Brom-phenyl)-(2,5-difluor-4-methoxy-phenyl)-methanon, (4-Brom-phenyl)-(2,5-difluor-4-hydroxy-phenyl)-methanon und (E)-[4-(4-Brom-but-2-enyloxy)-2,5-difluor-phenyl]-(4-brom-phenyl)-methanon.

### Beispiel 32

Analog 31 Beispiel erhält man
a) aus [4-[6-(Allyl-methyl-amino)-hexyloxy]-2-fluor-phenyl]-(4-brom-phenyl)-methanon das Allyl-[6-[3-(4-brom-phenyl)-1-methyl-1H-indazol-6-yloxy]-hexyl]-methyl-amin·Fumarat (1:1), MS: m/e 456 (M+H⁺, 1Br),
b) aus (E)-[4-[4-(Allyl-methyl-amino)-but-2-enyloxy]-3-fluor-phenyl]-(2,6-difluor-phenyl)-methanon das (E)-Allyl-[4-[2-fluor-4-(4-fluor-1-methyl-1H-indazol-3-yl)-phenoxy]-but-2-enyl]-methyl-amin·Fumarat (1:1), Smp. 108-111°C,
c) aus (E)-[4-[4-(Allyl-methyl-amino)-but-2-enyloxy]-2-fluor-phenyl]-(4-brom-phenyl)-methanon das (E)-Allyl-[4-[3-(4-brom-phenyl)-1-methyl-1H-indazol-6-yloxy]-but-2-enyl]-methyl-amin·Fumarat (1:1),Smp. 50-60 °C (Zersetztung), MS: m/e 426 (M+H⁺, 1Br),
d) aus [4-[6-(Allyl-methyl-amino)-hexyloxy]-phenyl]-(4-brom-2-fluor-phenyl)-methanon (Bsp Ad) das Allyl-[6-[4-(6-brom-1-methyl-1H-indazol-3-yl)-phenoxy]-hexyl]-methyl-amin·Fumarat (1:1), Smp. 152-154 °C (Zersetztung),
e) aus 1-[4-[6-(Allyl-methyl-amino)-hexyloxy]-2-fluor-pheny]-5-methyl-hex-5-en-1-on das Allyl-methyl-[6-[1-methyl-3-(4-methyl-pent-3-enyl)-1H-indazol-6-yloxy]-hexyl]-amin·Fumarat (1:1), MS: m/e 383 (M),
f) aus 1-[4-[6-(Allyl-methyl-amino)-hexyloxy]-2-fluor-pheny]-ethanon (Bsp Ae) das Allyl-[6-(1,3-dimethyl-1H-indazol-6-yloxy)-hexyl]-methyl-amin·Fumarat (1:1), MS: m/e 315 (M),
g) aus (E)-1-[4-[4-(Allyl-methyl-amino)-but-2-enyloxy]-2-fluor-phenyl]-ethanon das (E)-Allyl-[4-(1,3-dimethyl-1H-indazol-6-yloxy)-but-2-enyl]-methyl-amin, MS: m/e 286 (M+H⁺),
h) aus (E)-1-[4-[(E)-4-(Allyl-methyl-amino)-but-2-enyloxy]-2-fluor-phenyl]-5-methyl-4-hexen-1-on das (E)-Allyl-methyl-[4-[1-methyl-3-(4-methyl-pent-3-enyl)-1H-indazol-6-yloxy]-but-2-enyl]-amin·Fumarat (1:1), MS: m/e 354 (M+H⁺),
i) aus (4-Brom-phenyl)-[4-[6-(cyclopropyl-methyl-amino)-hexyloxy]-2-fluor-phenyl]-methanon das [6-[3-(4-Brom-phenyl)-1-methyl-1H-indazol-6-yloxy]-hexyl]-cyclopropyl-methyl-amin·Fumarat (1:1), Smp. 141-143 °C,
j) aus 1-[4-[6-(Cyclopropyl-methyl-amino)-hexyloxy]-2-fluor-phenyl]-5-methyl-hex-4-en-1-on das Cyclopropyl-methyl-[6-[3-(4-methyl-pent-3-enyl)-1H-indazol-6-yloxy]-hexyl]-amin·Fumarat (1:1), Smp. 78-79 °C,
k) aus [4-[6-(Allyl-methyl-amino)-hexyloxy]-2-fluor-phenyl]-(4-brom-phenyl)-methanon mit Hydrazin.Hydrat in DMSO das Allyl-[6-[3-(4-brom-phenyl)-1H-indazol-6-yloxy]-hexyl]-methyl-amin·Fumarat, Smp. 162-166 °C.

Ausgangsmaterialien.

Aus 1-(2-Fluor-4-hydroxy-phenyl)-5-methyl-hex-4-en-1-on, 1,6-Dibrom-hexan und N-Allyl-methyl-amin erhält man das 1-[4-[6-(Allyl-methyl-amino)-hexyloxy]-2-fluor-phenyl-5-methyl-hex-5-en-1-on, MS: m/e 375 (M), das Ausgangsmaterial zum Beispiel 32e).

Aus 2-Fluor-4-hydroxy-acetophenon, (E)-1,4-Dibrom-2-buten und N-Allyl-methyl-amin erhält man das (E)-1-[4-[4-(Allyl-methyl-amino)-but-2-enyloxy]-2-fluor-phenyl]-ethanon, MS: m/e 278 (M+H⁺), das Ausgangsmaterial zum Beispiel 32g).

Aus [4-(6-Brom-hexyloxy)-2-fluor-phenyl]-(4-brom-phenyl)-methanon und N-Methylcyclopropylamin·Hydrochlorid erhält man das (4-Brom-phenyl)-[4-[6-(cyclopropyl-methyl-amino)-hexyloxy]-2-fluor-phenyl]-methanon, MS: m/e 447 (M, 1Br), das Ausgangsmaterial zum Beispiel 32i).

Aus 1-(2-Fluor-4-hydroxy-phenyl)-5-methyl-hex-4-en-1-on, 1,6-Dibrom-hexan und Cyclopropyl-methylamin·Hydrochlorid erhält man das 1-[4-[6-(Cyclopropyl-methyl-amino)-hexyloxy]-2-fluor-phenyl]-5-methyl-hex-4-en-1-on, MS: m/e 376 (M+H⁺), das Ausgangsmaterial zum Beispiel 32j).

### Beispiel 33

### Allyl-[6-[4-(4-brom-phenyl)-quinazolin-7-yloxy]-hexyl]-methyl-amin

Eine Lösung von 230 mg [2-Amino-4-[6-(allyl-methyl-amino)-hexyloxy]-phenyl]-(4-brom-phenyl)-methanon in 0.5 ml Ameisensäure und 2 ml Formamid wird 25 Min bei 165 °C gekocht, dann eingeengt und mit Methylenchlorid / gesättigter Natriumbicarbonatlösung in das freie Amin (Titelverbindung) überführt. Nach der Reinigung (Kieselgel, Methylenchlorid/Methanol 2.5% bis 10%) wird der Rückstand in Methylenchlorid/Äther gelöst und mit 23.7 mg Fumarsäure versetzt. Nach Rühren und Abfiltrieren werden 30 mg Allyl-[6-[4-(4-brom-phenyl)-quinazolin-7-yloxy]-hexyl]-methyl-amin·Fumarat (1:1) erhalten, Smp 90-95 °C, MS: m/e 454 (M+H⁺, 1Br).

Ausgangsmaterial.

Es werden 17.55 g [4-[6-(Allyl-methyl-amino)-hexyloxy]-2-fluor-phenyl]-(4-brom-phenyl)-methanon mit 50.7 ml 4-Methoxybenzylamin und 6.5 g Kaliumcarbonat in 600 ml Toluol 23 Std unter Rückfluss gekocht. Nach Abfiltrieren, Eindampfen und Reinigung über Kieselgel mit Methylenchlorid/Methanol (2.5% bis 10%) werden 17.43 g [4-[6-(Allyl-methyl-amino)-hexyloxy]-2-[(4-methoxy-benzyl)amino]-phenyl]-(4-brom-phenyl)-methanon erhalten. Eine Lösung von diesem Material in 200 ml Trifluoressigsäure wird während 45 Std bei Raumtemperatur gerührt, eingedampft und mit Methylenchlorid/gesättigter Natriumbicarbonat-Lösung in die frei Base überführt. Nach Reinigung über Kieselgel mit Methylenchlorid/Methanol (9:1) werden 13.23 g [2-Amino-4-[6-(allyl-methyl-amino)-hexyloxy]-phenyl]-(4-brom-phenyl)-methanon, Smp. des Fumarats 78°C (Zersetzung), erhalten.

### Beispiel 34

### (RS)-Allyl-[6-[4-(4-brom-phenyl)-3,4-dihydro-quinazolin-7-yloxy]-hexyl]-methyl-amin

Eine Lösung von 445 mg [2-Amino-4-[6-(allyl-methyl-amino)-hexyloxy]-phenyl]-(4-brom-phenyl)-methanon in 1 ml Ameisensäure und 4 ml Formamid wird 35 Std bei 160 °C gekocht, dann eingeengt und mit Methylenchlorid gesättigter Natriumbicarbonatlösung ins freie Amin (Titelverbindung) überführt. Der Rückstand wird in Methylenchlorid/Äther gelöst und mit 73 mg Fumarsäure versetzt. Nach Rühren wird abdekantiert, der Rückstand mit Äther verrieben und abfiltriert. Es werden 30 mg (RS)-Allyl-[6-[4-(4-brom-phenyl)-3,4-dihydro-quinazolin-7-yloxy]-hexyl]-methyl-amin·Fumarat (1:1), Smp 120 °C (Zersetztung) erhalten, MS: m/e 456 (M+H⁺, 1Br).

### Beispiel 35

### 7-[6-(Allyl-methyl-amino)-hexyloxy]-4-(4-brom-phenyl)-1-methyl-1H-quinazolin-2-on

Eine Lösung von 0.16 ml Chlorsulfonylisocyanat in 0.8 ml Methylenchlorid wird bei 0 °C zu einer Lösung von 0.69 g [4-[6-(Allyl-methyl-amino)-hexyloxy]-2-methylamino-phenyl]-(4-brom-phenyl)-methanon in 4 ml Methylenchlorid getropft. Nach 3 Std bei Raumtemperatur wird erneut auf 0 °C abgekühlt und 0.8 ml Chlorsulfonylisocyanat in 0.5 ml Methylenchlorid werden zugetropft. Nach 1 Std bei Raumtemperatur wird mit gesättigter Natriumbicarbonat-Lösung/Methylenchlorid aufgearbeitet und über Kieselgel mit Methylenchlorid/Methanol/25%-iger wässriger Ammoniumhydroxid (95:5:0.5) gereinigt. Es werden 0.26 g 7-[6-(Allyl-methyl-amino)-hexyloxy]-4-(4-brom-phenyl)-1-methyl-1H-quinazolin-2-on erhalten, MS: m/e 484 (M+H⁺, 1Br).

Ausgangsmaterial.

Eine Lösung von 2.6 g [4-[6-(Allyl-methyl-amino)-hexyloxy]-2-fluor-phenyl]-(4-brom-phenyl)-methanon in 115 ml Dimethylacetamid wird mit 7.2 ml 8.03M Methylamin in Ethanol 3 Std bei 120°C gekocht, eingeengt und über Kieselgel mit Methylenchlorid/Methanol (2.5%-10%) chromatographiert. Man erhält das [4-[6-(Allyl-methyl-amino)-hexyloxy]-2-methylamino-phenyl]-(4-brom-phenyl)-methanon, Smp. des Fumarats 72°C.

### Beispiel 36

### Allyl-[6-[4-(4-brom-phenyl)-2-methyl-quinolin-7-yloxy]-hexyl]-methyl-amin

Eine Lösung von 360 mg [2-Amino-4-[6-(allyl-methyl-amino)-hexyloxy]-phenyl]-(4-brom-phenyl)-methanon in 1.6 ml Essigsäure wird mit 0.01 ml H₂SO₄ und 0.4 ml Aceton versetzt und 21 Std bei 95 °C gekocht, wobei nach 6 Std noch einmal 0.4 ml Aceton zugegeben werden. Bei 0 °C wird die Reaktion in eine eiskalte Lösung von 1.2 ml 25%-iger Ammoniumhydroxidlösung in 3.5 ml Wasser gegossen und anschliessend mit Methylenchlorid extrahiert. Nach dem Trocknen der organischen Phase wird eingedampft und der Rückstand über Kieselgel mit Methylenchlorid/ Methanol (95:5) gereinigt. Die erhaltene Titelverbindung (200 mg) wird mit 24.6 mg Fumarsäure aus Methylenchlorid/Methanol/Äther kristallisiert. Man erhält 56 mg Allyl-[6-[4-(4-brom-phenyl)-2-methyl-quinolin-7-yloxy]-hexyl]-methyl-amin·Fumarat (1:2), Smp 88-93 °C, MS: m/e 467 (M+H⁺, 1Br).

### Beispiel 37

### Allyl-[6-[4-(4-brom-phenyl)-2H-chromen-7-yloxy]-hexyl]-methyl-amin

Eine Lösung von 0.26 g (RS)-5-[6-(Allyl-methyl-amino)-hexyloxy]-2-[1-(4-brom-phenyl)-1-hydroxy-allyl]-phenol in 50 ml o-Xylol wird 2 Std bei 170 °C am Wasserabscheider gekocht, eingedampft und über Kieselgel mit Methylenchlorid/Methanol 95:5 gereinigt. Man erhält 0.18 g Allyl-[6-[4-(4-brom-phenyl)-2H-chromen-7-yloxy]-hexyl]-methyl-amin, MS: m/e 456 (M+H⁺, 1Br).

Ausgangsmaterial.

Es werden 8.97 g [4-[6-(Allyl-methyl-amino)-hexyloxy]-2-fluor-phenyl]-(4-brom-phenyl)-methanon in 450 ml THF mit 37 ml 5.4M Natrium-methanolat in Methanol 14 Std bei Raumtemperatur und 1 Std unter Rückfluss gerührt. Die Lösung wird eingedampft und in Methylenchlorid/10%iger Natriumchlorid-Lösung aufgenommen. Die organische Phase wird getrocknet, in Äther gelöst und mit 2.08 g Fumarsäure über Nacht gerührt. Man erhält 8.17 g [4-[6-(Allyl-methyl-amino)-hexyloxy]-2-methoxy-phenyl]-(4-brom-phenyl)-methanon·Fumarat (Smp. 108-113 °C).

Das erhaltene Fumarat wird in Methylenchlorid/gesättigter Natriumbicarbonat-Lösung aufgenommen, die organische Phase wird getrocknet und eingeengt. 3.09 g des so erhaltenen [4-[6-(Allyl-methyl-amino)-hexyloxy]-2-methoxy-phenyl]-(4-brom-phenyl)-methanons werden in 13 ml Essigsäure/7.7 ml 62%-iger wässriger Bromwasserstofflösung 2 Std bei 90 °C gekocht. Das Reaktionsgemisch wird eingeengt und mit Methylenchlorid/gesättigter Natriumbicarbonat-Lösung in die freie Base umgewandelt. Der Rückstand enthält das [4-[6-(Allyl-methyl-amino)-hexyloxy]-2-hydroxy-phenyl]-(4-brom-phenyl)-methanon, MS: m/e 446 (M+H⁺, 1 Br).

Zu 4.8 ml (1.7 M in THF) Vinylmagnesiumchlorid-Lösung werden bei 0°C 1.0g [4-[6-(Allyl-methyl-amino)-hexyloxy]-2-hydroxy-phenyl]-(4-bromo-phenyl)-methanon in 9 ml THF/Äther (1:1) während 45 Min getropft. Die Reaktionslösung wird über Nacht auf Raumtemperatur aufgewärmt, mit 3 ml Essigsäure/Wasser (1:1) versetzt und mit gesättigter Natriumbicarbonat-Lösung/Methylenchlorid aufgearbeitet. Nach dem Trocknen der organischen Phase wird eingeengt und über Kieselgel mit Methylenchlorid/Methanol (95:5) gereinigt. Man erhält 0.64 g (RS)-5-[6-(Allyl-methyl-amino)-hexyloxy]-2-[1-(4-brom-phenyl)-1-hydroxy-allyl]-phenol, MS: m/e 474 (M+H⁺, 1Br).

### Beispiel 38

### Allyl-[6-[4-(4-brom-phenyl)-quinolin-7-yloxy]-hexyl]-methyl-amin

Eine Lösung von 0.5 g (RS)-1-[4-[6-(Allyl-methyl-amino)-hexyloxy]-2-amino-phenyl]-1-(4-brom-phenyl)-prop-2-en-1-ol wird bei 0°C innerhalb von 10 Min zu einer Suspension von 0.25 g Pyridinium-chlorochromat in Methylenchlorid getropft. Nach 1.5 Std bei Raumtemperatur wird Wasser zugegeben, mit 2 M Natriumhydroxid auf pH 12 gestellt und mit Methylenchlorid extrahiert. Die organische Phase wird getrocknet, eingeengt und über Kieselgel mit Methylenchlorid/Methanol 95:5 als Eluens gereinigt. Es werden 80 mg der Titelverbindung erhalten. 53 mg davon werden in Methylenchlorid/Essigester gelöst und mit 26 mg Fumarsäure gerührt. Nach Fitration erhält man Allyl-[6-[4-(4-brom-phenyl)-quinolin-7-yloxy]-hexyl]-methyl-amin·Fumarat (1:3), Smp 70°C, MS: m/e 453 (M+H⁺, 1Br).

Das Ausgangsmaterial, (RS)-1-[4-[6-(Allyl-methyl-amino)-hexyloxy]-2-amino-phenyl]-1-(4-brom-phenyl)-prop-2-en-1-ol, MS: m/e 473 (M+H⁺, 1Br), erhält man durch Reaktion von Vinylmagnesiumchlorid mit [2-Amino-4-[6-(allyl-methyl-amino)-hexyloxy]-phenyl]-(4-brom-phenyl)-methanon.

### Beispiel 39

### (1RS,2RS)-[2-[1-(4-Brom-phenyl)-isoquinolin-6-yloxymethyl]-cyclopropyl-methyl]-cyclopropyl-methyl-amin

Eine Lösung von 350 mg Triphenylphosphin, 200 mg 1-(4-Brom-phenyl)-isoquinolin-6-ol (Bsp 23b) und 103.4 mg (1RS,2RS)-[2-[(Cyclopropyl-methyl-amino)-methyl]-cyclopropyl]-methanol in 5.4 ml THF wird bei Raumtemperatur innerhalb 1 Std mit 0.22 ml Diäthylazodicarboxylat in 0.5 ml THF behandelt. Nach 16 Std Nachrühren wird eingeegut. Der Rückstand wird in Äther gelöst und mit Hexan gefällt. Die Mutterlauge wird eingeengt und über Kieselgel mit Essigester als Eluens gereinigt. Die erhaltenen 75 mg der Titelverbindung werden in Methylenchlorid/Methanol mit 17.9 mg Fumarsäure versetzt und mit Essigester/Äther gefällt. Man erhält 33 mg (1RS,2RS)-[2-[1-(4-Brom-phenyl)-isoquinolin-6-yloxymethyl]-cyclopropyl-methyl]-cyclopropyl-methyl-amin·Fumarate (1:1), MS: m/e 437 (M+H⁺, 1Br).

Ausgangsverbindung

Zu einer Lösung von 5.0 g (1RS,2RS)-1,2-Cyclopropandicarbonsäure-diäthylester in 9 ml Methanol werden 13.4 ml 2 M Kaliumhydroxid in Methanol zugetropft. Nach 2.5 Std wird mit 8%-iger Phosphorsäure angesäuert und mit gesättigter Natriumchloridlösung/Methylenchlorid extrahiert, getrocknet und eingeengt wobei man 5.1 g (1RS,2RS)-1,2-Cyclopropandicarbonsäure-monomethylester erhält.

Eine Lösung von 9.3 g (1RS,2RS)-1,2-Cyclopropandicarbonsäure-monomethylester, 4.0 ml N-Cyclopropylamin und 11.6 g N-(3-Dimethyl-aminopropyl)-N'-äthylcarbodiimid-hydrochlorid in 210 ml Methylenchlorid wird bei 0 °C mit 0.7 g Dimethylaminopyridin versetzt und anschliessend 2 Std bei Raumtemperatur gerührt. Die Reaktionslösung wird mit Methylenchlorid/10%-iger Kaliumhydrogensulfatlösung aufgearbeitet. Die organische Phase wird mit gesättigter Natriumbicarbonatlösung gewaschen, getrocknet und eingeengt, wobei man 10.6 g (1RS,2RS)-2-Cyclopropylcarbamoyl-cyclopropancarbonsäure-methylester erhält.

Eine Lösung von 4.9 g (1RS,2RS)-2-Cyclopropylcarbamoyl-cyclopropan-carbonsäure-methylester und 10.8 ml Methyliodid in 120 ml 1,2-Dimethoxyäthan wird bei 0 °C mit 1.2 g 55% igem Natriumhydrid behandelt und während 22 Std bei 0 °C gerührt. Nach Zugabe von Wasser wird eingedampft und mit 10%-iger Kaliumhydrogensulfatlösung/Äther extrahiert, mit gesättigter Natriumchloridlösung gewaschen und die organische Phase getrocknet.

Der rohe (1RS,2RS)-2-(Cyclopropyl-methyl-carbamoyl)-cyclopropan-carbonsäure-methylester wird in 9 ml THF gelöst und in eine siedende Suspension von 1.4g Lithiumaluminiumhydrid in 40 ml THF getropft. Das Reaktionsgemisch wird während weiteren 24 Std gekocht, dannauf 0 °C gekühlt und mit 9 ml Wasser versetzt, getrocknet, abfiltriert und eingeengt. Das erhaltene Öl wird in Methylenchlorid gelöst, getrocknet und eingeengt, wobei man 4.2 g (1RS,2RS)-[2-[(Cylcopropyl-methyl-amino)-methyl]-cyclopropyl]-methanol erhält, MS: m/e 156 (M+H⁺).

Pharmazeutische Anwendungsformen folgender Zusammensetzung können in an sich bekannter Weise hergestellt werden:

### Beispiel a

### Tabletten mit 5 mg Allyl-[6-[1-(4-brom-phenyl)-isoquinolin-6-yloxy]-hexyl]-methyl-amin als Wirkstoff

| Zusammensetzung: | 1 Tablette enthält: |
|---|---|
| Wirkstoff | 5,0 mg |
| Milchzucker | 148,0 mg |
| Kartoffelstärke | 65,0 mg |
| Magnesiumstearat | 2,0 mg |
| | 220,0 mg |

### Beispiel b

### Dragées mit 5 mg Allyl-[6-[1-(4-brom-phenyl)-isoquinolin-6-yloxy]-hexyl]-methyl-amin

Die Tabletten von Bsp. A werden nach bekanntem Verfahren mit einer Hülle überzogen, die im wesentlichen aus Zucker und Talkum besteht. Die fertigen Dragées werden mit Hilfe von Bienenwachs poliert.

| | |
|---|---|
| Dragéegewicht: | 300 mg |

### Beispiel c

### Suppositorien mit 5 mg Allyl-[6-[1-(4-brom-phenyl)-isoquinolin-6-yloxy]-hexyl]-methyl-amin als Wirkstoff

| Zusammensetzung: | 1 Zäpfchen enthält: |
|---|---|
| Wirkstoff | 5,0 mg |
| Zäpfchenmasse (z.B. Witepsol W 45®) | 1695,0 mg |
| | 1700,0 mg |

### Beispiel d

### Kapseln mit 5 mg Allyl-[6-[1-(4-brom-phenyl)-isoquinolin-6-yloxy]-hexyl]-methyl-amin als Wirkstoff

| Zusammensetzung: | 1 Kapsel enthält: |
|---|---|
| Wirkstoff | 5,0 mg |
| Lactose | 82,0 mg |
| Stärke | 82,0 mg |
| Magnesiumstearat | 1,0 mg |
| | 170,0 mg |

## Patentansprüche

1. Aminoalkyl-substituierte benzo-heterocyclische Verbindungen der Formel 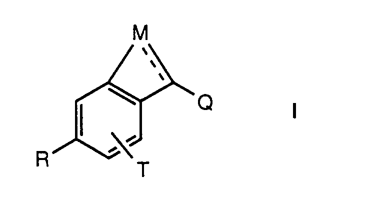 worin
R entweder eine Gruppe der Formel 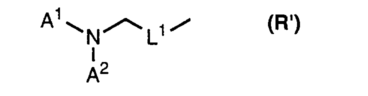
T H, Alkyl, Halogen, N(R²,R²¹), CONH₂, CN, NO₂, CF₃, OH oder Alkyl (O oder S),
R² und R²¹ Alkyl oder H und
Q Cycloalkyl, durch R³ und R³¹ substituiertes Phenyl oder eine gegebenenfalls durch OH substituierte Alkenyl- oder Alkadienylgruppe mit bis zu 13 C-Atomen oder, falls M nicht durch ein O-Atom unterbrochen ist, dann Q auch gegebenenfalls durch OH substituiertes C₁₋₁₃-Alkyl sein kann,
oder worin eins von R und T Halogen oder H und das andere H, Alkyl, Halogen, N(R²,R²¹), CONH₂, CN, NO₂, CF₃, OH oder Alkyl (O oder S) und
Q eine Gruppe der Formel 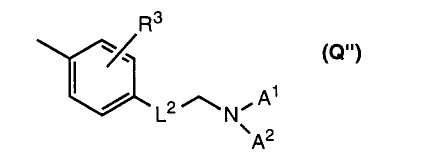
A¹ Alkyl oder Alkenyl und
A² Cycloalkyl, Cycloalkyl-alkyl oder eine gegebenenfalls durch R⁴ substituierte Alkyl- oder Alkenylgruppe oder
A¹ zusammen mit A² eine gegebenenfalls durch R⁴ substituierte Alkylen- oder Alkenylengruppe A¹-A² mit bis zu 5 C-Atomen bildet,
R⁴ an einem gesättigten C-Atom von A² oder von A¹-A² gebundenes OH oder Alkyl (O oder S), wobei ein durch R⁴ substituiertes C-Atom bzw. ein in A¹, A² oder A¹-A² enthaltenes ungesättigtes C-Atom in einer anderen als der α-Stellung zu N(A¹A²) gebunden sein soll,
L¹ eine direkt oder über O, NH oder N(Alkyl oder Alkanoyl) an die Benzogruppe gebundene Gruppe L,
L² eine über O, NH oder N(Alkyl oder Alkanoyl) an die Phenylgruppe gebundene Gruppe L,
L eine Alkylen- oder Alkenylengruppe mit insgesamt bis zu 11 C-Atomen und zumindest 4 bzw. 3 C-Atomen zwischen den zwei freien Valenzen oder eine (C₃₋₆-Cycloalkylen)-alkylengruppe, die über die Cycloalkylengruppe an die Methylengruppe gebunden ist,
R³ und R³¹ H, Alkyl, Halogen, N(R⁵,R⁵¹), CONH₂, CN, NO₂, CF₃, OH oder Alkyl (O oder S),
R⁵ und R⁵¹ Alkyl oder H,
die von M ausgehende gestrichelte Bindung fakultativ ist,
M eine 2- oder 3-gliedrige Gruppierung, die ein O- oder S-Atom oder eine Gruppe S(O), S(O)₂ oder C(O) und/oder bis zu zwei N-Atome oder Gruppen N(R⁶) und/oder bis zu zwei Gruppen C(R⁶) oder CH(R⁶) enthalten kann, jedoch insgesamt zumindest ein gegebenenfalls substituiertes Heteroatom enthält und
R⁶ H oder Alkyl ist,
und Säureadditionssalze davon.

2. Verbindungen nach Anspruch 1 und der Formel 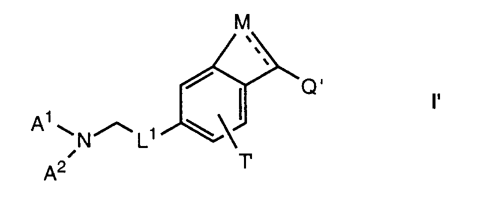 worin
T' H, Alkyl, Halogen, N(R²,R²¹), CONH₂, CN, NO₂, CF₃, OH oder Alkyl (O oder S),
Q' Cycloalkyl, durch R³ und R³¹ substituiertes Phenyl oder eine gegebenenfalls durch OH substituierte Alkenyl- oder AIkadienylgruppe mit bis zu 13 C-Atomen oder, falls M nicht durch ein O-Atom unterbrochen ist, dann Q auch gegebenenfalls durch OH substituiertes C₁₋₁₃-Alkyl sein kann,
L¹ eine direkt oder über O, NH oder N(Alkyl oder Alkanoyl) an die Benzogruppe gebundene Gruppe L ist und
A¹, A², L, M, R², R²¹, R³ und R³¹ die gleiche Bedeutung wie in Anspruch 1 haben.

3. Verbindungen nach Anspruch 1 und der Formel 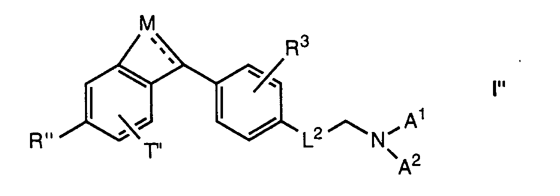 worin
eins von R'' und T'' Halogen oder H und das andere H, Alkyl, Halogen, N(R²,R²¹), CONH₂, CN, NO₂, CF₃, OH oder Alkyl (O oder S),
L² eine über O, NH oder N(Alkyl oder Alkanoyl) an die Phenylgruppe gebundene Gruppe L ist und
A¹, A², L, M, R², R²¹ und R³ die gleiche Bedeutung wie im Anspruch 1 haben.

4. Verbindungen nach Anspruch 1 oder 2 und der Formel 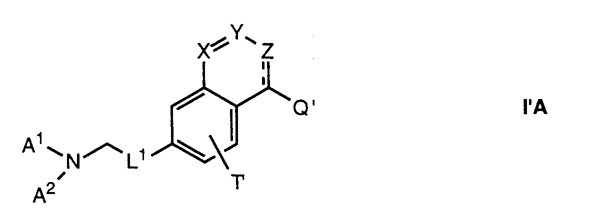 worin die Gruppierung X=Y-Z ein oder zwei N-Atome und zwei bzw. eine Gruppe CH oder C(Alkyl) enthält,
insbesondere worin X=Y-Z eine Gruppierung CH=CH-N, CH=N-N, N=CH-N, N=CH-NH, N=CH-CH oder N=C(Alkyl)-CH, speziell N=C(CH₃)-CH ist.

5. Verbindungen nach Anspruch 1 oder 2 und der Formel 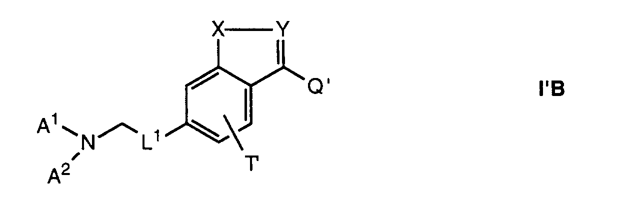 worin X ein O- oder S-Atom oder eine Gruppe SO₂, NH oder N(Alkyl), insbesondere N(CH₃), und Y ein N-Atom oder CH ist.

6. Verbindungen nach Anspruch 1 oder 2 und der Formel 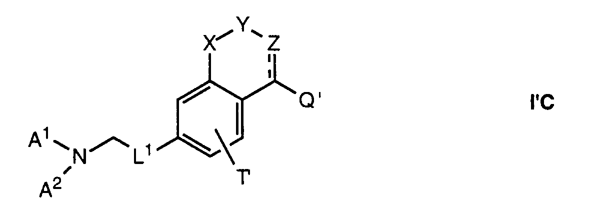 worin die Gruppierung X-Y-Z ein O-Atom oder eine Gruppe C(O) und/oder bis zu zwei N-Atome oder Gruppen N(R⁶) und/oder bis zu zwei Gruppen C(R⁶) oder CH(R⁶) enthalten kann, jedoch insgesamt ein oder zwei gegebenenfalls substituierte Heteroatome enthält, insbesondere worin X-Y-Z eine Gruppierung CH₂-O-N, O-CH₂-CH, N(R⁶)-C(O)-N, insbesondere N(CH₃)-C(O)-N, CH₂-CH₂-N oder CH₂-CH₂-N(R⁶), insbesondere CH₂-CH₂-NH oder CH₂-CH₂-N(CH₃) ist.

7. Verbindungen nach Anspruch 1 oder 3 und der Formel 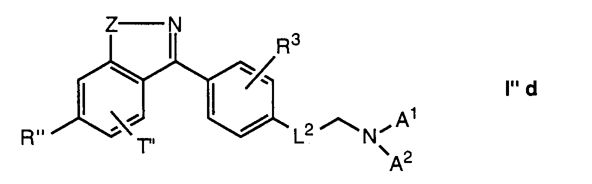 worin Z ein O- oder S-Atom oder eine Gruppe SO₂ oder N(Alkyl), insbesondere N-Methyl, oder worin Z zusammen mit N die Gruppe CH₂-O-N ist.

8. Verbindungen nach Anspruch 4, worin A¹ Alkyl, A² Alkenyl oder Cycloalkyl, L¹ eine über ein O-Atom an die Benzogruppe gebundene Gruppe L, T' Wasserstoff und Q' durch R³ substituiertes Phenyl ist.

9. Verbindungen nach Anspruch 5, worin A¹ Alkyl, A² Alkenyl, Cycloalkyl oder Alkyl-S-alkyl, L¹ eine über ein O-Atom an die Benzogruppe gebundene Alkylen- oder Alkenylengruppe mit insgesamt bis zu 11 C-Atomen und zumindest 4 bzw. 3 C-Atomen zwischen den zwei freien Valenzen, T' Wasserstoff oder Halogen und Q' durch R³ substituiertes Phenyl oder eine Alkyl- oder gegebenenfalls durch OH substituierte Alkenyl- oder Alkadienylgruppe mit bis zu 13 C-Atomen.

10. Verbindungen nach Anspruch 6, worin A¹ Alkyl, A² Alkenyl, L¹ eine über ein O-Atom an die Benzogruppe gebundene Alkylen- oder Alkenylengruppe mit insgesamt bis zu 11 C-Atomen und zumindest 4 bzw. 3 C-Atomen zwischen den zwei freien Valenzen, T' Wasserstoff und Q' durch R³ substituiertes Phenyl ist.

11. Verbindungen nach Anspruch 7, worin A¹ Alkyl, A² Alkenyl, L² über ein O-Atom an die Phenylgruppe gebundene Alkylen- oder Alkenylengruppe mit insgesamt bis zu 11 C-Atomen und zumindest 4 bzw. 3 C-Atomen zwischen den freien Valenzen, R³ Wasserstoff und R³¹, R'' und T'' Wasserstoff oder Halogen sind.

12. Verbindungen nach Anspruch 4 oder 8, worin X=Y-Z eine Gruppierung CH=CH-N, CH=N-N, N=CH-N oder N=CH-CH, insbesondere worin A¹ Methyl, A² Allyl oder Cyclopropyl, L¹ n-Pentylenoxy oder Cyclopropylenmethylenoxy, T' Wasserstoff und durch Q' Brom substituiertes Phenyl ist, speziell die Verbindungen:
Allyl-[6-[1-(4-brom-phenyl)-isoquinolin-6-yloxy]-hexyl]-methyl-amin
[6-[1-(4-Brom-phenyl)-isoquinolin-6-yloxy]-hexyl]-cyclopropyl-methyl-amin,
Allyl-[6-[1-(4-brom-phenyl)-phthalazin-6-yloxy]-hexyl]-methyl-amin
Allyl-[6-[4-(4-brom-phenyl)-quinazolin-7-yloxy]-hexyl]-methyl-amin
Allyl-[6-[4-(4-brom-phenyl)-quinolin-7-yloxy]-hexyl]-methyl-amin
(1RS,2RS)-[2-[1-(4-Brom-phenyl)-isoquinolin-6-yloxymethyl]-cyclopropyl-methyl]-cyclopropyl-methyl-amin.

13. Verbindungen nach Anspruch 4 oder 8:
(E)-Allyl-[4-[1-(4-brom-phenyl)-isoquinolin-6-yloxy]-but-2-enyl]-methyl-amin
(E)-Allyl-[4-[1-(4-brom-phenyl)-phthalazin-6-yloxy]-but-2-enyl]-methyl-amin
Allyl-[6-[4-(4-brom-phenyl)-2-methyl-quinolin-7-yloxy]-hexyl]-methyl-amin.

14. Verbindungen nach Anspruch 5 oder 9, worin X-Y eine Gruppe S-CH, O-N, S-N, NH-N oder N(CH₃)-N, insbesondere worin A¹ Methyl, A² Allyl, Cyclopropyl oder Methylsulfonylethyl, L¹ n-Pentylenoxy oder n-Propenylenoxy, Q' Bromophenyl oder 4-Methyl-pent-3-enyl und T' Wasserstoff oder Fluor ist, speziell die Verbindungen:
Allyl-[6-[3-(4-brom-phenyl)-benzo[d]isothiazol-6-yloxy]-hexyl]-methyl-amin
6-[3-(4-Brom-phenyl)-benzo[d]isothiazol-6-yloxy]-hexyl]-methyl-(2-methylsulfanyl-ethyl)-amin
Allyl-[6-[3-(4-brom-phenyl)-benzo[b]thiophen-6-yloxy]-hexyl]-methyl-amin
(E)-Allyl-methyl-[4-[3-(4-methyl-pent-3-enyl)-benzo[d]isothiazol-6-yloxy]-but-2-enyl]-amin
(E)-Allyl-[4-[3-(4-brom-phenyl)-benzo[d]isoxazol-6-yloxy]-but-2-enyl-methyl-amin
(E)-Allyl-[4-[3-(4-brom-phenyl)-5-fluor-1-methyl-1H-indazol-6-yloxy]-but-2-enyl]-methyl-amin
Allyl-methyl-[6-[1-methyl-3-(4-methyl-pent-3-enyl)-1H-indazol-6-yloxy]-hexyl]-amin
(E)-Allyl-methyl-[4-[1-methyl-3-(4-methyl-pent-3-enyl)-1H-indazol-6-yloxy]-but-2-enyl]-amin
[6-[3-(4-Brom-phenyl)-1-methyl-1H-indazol-6-yloxy]-hexyl]-cyclopropyl-methyl-amin
Allyl-[6-[3-(4-brom-phenyl)-1H-indazol-6-yloxy]-hexyl]-methyl-amin.

15. Verbindungen nach Anspruch 5 oder 9:
(E)-Allyl-[4-[3-(4-brom-phenyl)-benzo[d]isothiazol-6-yloxy]-but-2-enyl]-methyl-amin
(E)-[4-[3-(4-Brom-phenyl)-benzo[d]isothiazol-6-yloxy]-but-2-enyl]-methyl-(2-methylsulfanyl-ethyl)-amin
Allyl-methyl-[6-(3-methyl-benzo[d]isothiazol-6-yloxy)-hexyl]-amin
(E)-Allyl-methyl-[4-(3-methyl-benzo[d]isothiazol-6-yloxy)-but-2-enyl]-amin
(E)-Allyl-[4-[3-(4-brom-phenyl)-benzo[b]thiopen-6-yloxy]-but-2-enyl]-methyl-amin
Allyl-methyl-[6-[3-(4-methyl-pent-3-enyl)-benzo[d]isothiazol-6-yloxy]-hexyl-amin
(E)-Allyl-methyl-[6-[3-(4-methyl-penta-1,3-dienyl)-benzo[d]isothiazol-6-yloxy]-hexyl]-amin
(RS)-1-[6-[6-(Allyl-methyl-amino)-hexyloxy]-benzo[d]isothiazol-3-yl]-4-methyl-pent-3-en-2-ol
(E)-(RS)-1-[6-[4-(Allyl-methyl-amino)-but-2-enyloxy]-benzo[d]isothiazol-3-yl]-4-methyl-pent-3-en-2-ol
Allyl-methyl-[(E)-4-[3-[(E)-4-methyl-penta-1,3-dienyl]-benzo[d]isothiazol-6-yloxy]-but-2-enyl]-amin
7:3 Gemisch von (E)-Allyl-methyl-[6-[3-(4-methyl-penta-2,4-dienyl)-benzo[d]isothiazol-6-yloxy]-hexyl]-amin und (E)-Allyl-methyl-[6-[3-(4-methyl-penta-1,3-dienyl)-benzo[d]isothiazol-6-yloxy]-hexyl]-amin
Allyl-[6-[4-(6-brom-1,1-dioxo-benzo[d]isothiazol-3-yl)-phenoxy]-hexyl]-methyl-amin
Allyl-[6-[3-(4-brom-phenyl)-benzo[d]isoxazol-6-yloxy]-hexyl]-methyl-amin
[6-[3-(4-Brom-phenyl)-benzo[d]isoxazol-6-yloxy]-hexyl]-cyclopropyl-methyl-amin
(E)-Allyl-methyl-[4-[3-(4-methyl-pent-3-enyl)-benzo[d]isoxazol-6-yloxy]-but-2-enyl]-amin
(E)-Allyl-[4-[3-(4-brom-phenyl)-benzofuran-6-yloxy]-but-2-enyl]-methyl-amin
Allyl-[6-[3-(4-brom-phenyl)-benzofuran-6-yloxy]-hexyl]-methyl-amin
Allyl-[6-[3-(4-brom-phenyl)-1-methyl-1H-indazol-6-yloxy]-hexyl]-methyl-amin
(E)-Allyl-[4-[3-(4-brom-phenyl)-1-methyl-1H-indazol-6-yloxy]-but-2-enyl]-methyl-amin
Allyl-[6-(1,3-dimethyl-1H-indazol-6-yloxy)-hexyl]-methyl-amin
(E)-Allyl-[4-(1,3-dimethyl-1H-indazol-6-yloxy)-but-2-enyl]-methyl-amin
Cyclopropyl-methyl-[6-[3-(4-methyl-pent-3-enyl)-1H-indazol-6-yloxy]-hexyl]-amin.

16. Verbindungen nach Anspruch 6 oder 10, worin X-Y-Z eine Gruppierung CH₂-O-N, CH₂-CH₂-N oder O-CH₂-CH, insbesondere worin A¹ Methyl, A² Allyl, L¹ n-Pentylenoxy oder n-Propenylenoxy, T' Wasserstoff und Q' Bromophenyl ist, speziell die Verbindungen:
(E)-Allyl-[4-[1-(4-brom-phenyl)-3,4-dihydro-isoquinolin-6-yloxy]-but-2-enyl]-methyl-amin
(E)-Allyl-[4-[4-(4-brom-phenyl)-1H-benzo[d][1,2]oxazin-7-yloxy]-but-2-enyl]-methyl-amin
Allyl-[6-[4-(4-brom-phenyl)-2H-chromen-7-yloxy]-hexyl]-methyl-amin.

17. Verbindungen nach Anspruch 6 oder 10:
Allyl-[6-[1-(4-brom-phenyl)-3,4-dihydro-isoquinolin-6-yloxy]-hexyl]-methyl-amin
(RS)-Allyl-[6-[1-(4-brom-phenyl)-1,2,3,4-tetrahydro-isoquinolin-6-yloxy]-hexyl]-methyl-amin
(RS)-Allyl-[6-[1-(4-brom-phenyl)-2-methyl-1,2,3,4-tetrahydro-isoquinolin-6-yloxy]-hexyl]-methyl-amin
Allyl-6-[4-(4-brom-phenyl)-1H-benzo[d][1,2]oxazin-7-yloxy]-hexyl-methyl-amin
7-[6-(Allyl-methyl-amino)-hexyloxy]-4-(4-brom-phenyl)-1-methyl-1H-quinazolin-2-on.

18. Verbindungen nach Anspruch 7 oder 11, worin Z ein O- oder S-Atom oder eine Gruppe SO₂ oder N-Methyl oder worin Z zusammen mit N die Gruppe CH₂-O-N, insbesondere worin A¹ Methyl, A² Allyl, L² n-Pentylenoxy oder n-Propylenoxy, R³ Wasserstoff, R³¹ Wasserstoff oder Fluor, R'' Wasserstoff oder Brom und T'' Wasserstoff oder Fluor ist, speziell die Verbindungen:
Allyl-[6-[4-(6-brom-benzo[d]isothiazol-3-yl)-phenoxy]-hexyl]-methyl-amin
(E)-Allyl-[4-[4-(6-brom-benzo[d]isothiazol-3-yl)-phenoxy]-but-2-enyl]-methyl-amin
Allyl-[6-[4-(6-brom-1,1-dioxo-benzo[d]isothiazol-3-yl)-phenoxy]-hexyl]-methyl-amin
Allyl-[6-[4-(6-brom-benzo[d]isoxazol-3-yl)-phenoxy]-hexyl-methyl-amin
(E)-Allyl-[4-[4-(6-brom-benzo[d]isoxazol-3-yl)-phenoxy]-but-2-enyl]-methyl-amin
Allyl-[6-[4-(7-brom-1H-benzo[d][1,2]oxazin-4-yl)-phenoxy]-hexyl]-methyl-amin
(E)-Allyl-[4-[4-(7-brom-1H-benzo[d][1,2]oxazin-4-yl)-phenoxy]-but-2-enyl-methyl-amin
(E)-Allyl-[4-[2-fluor-4-(4-fluor-1-methyl-1H-indazol-3-yl)-phenoxy]-but-2-enyl]-methyl-amin
Allyl-[6-[4-(6-brom-1-methyl-1H-indazol-3-yl)-phenoxy]-hexyl]-methyl-amin.

19. Verbindungen nach einem der Ansprüche 1-18 zur Verwendung als therapeutische Wirkstoffe.

20. Verfahren zur Herstellung der Verbindungen nach Anspruch 1, dadurch gekennzeichnet, dass man
a) ein Bromid der Formel 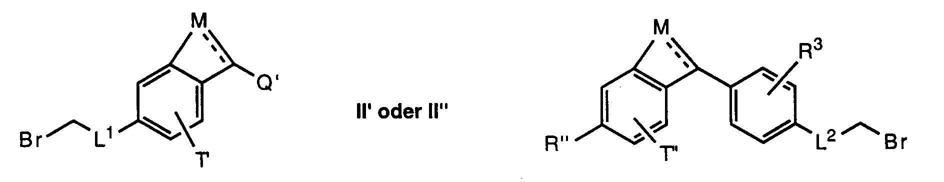 mit einem Amin HN(A¹A²) umsetzt,
b) ein Keton der Formel 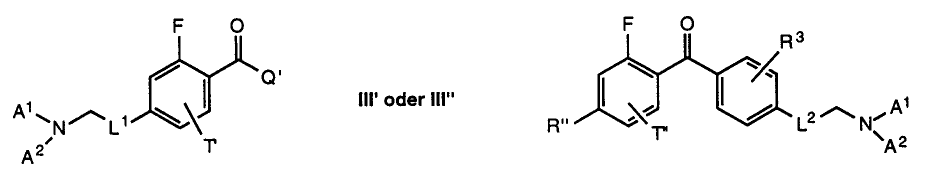 mittels H₂NNH-R⁶ cyclisiert,
c) ein Oxim der Formel 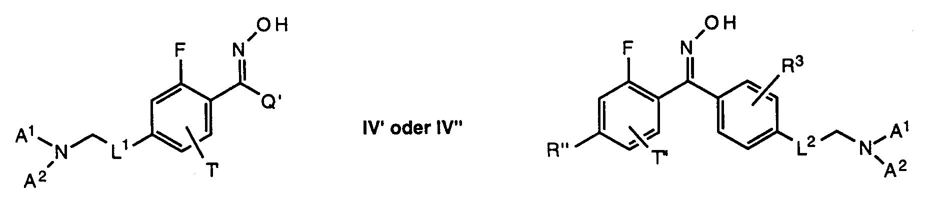 cyclisiert,
d) ein Aldehyd der Formel 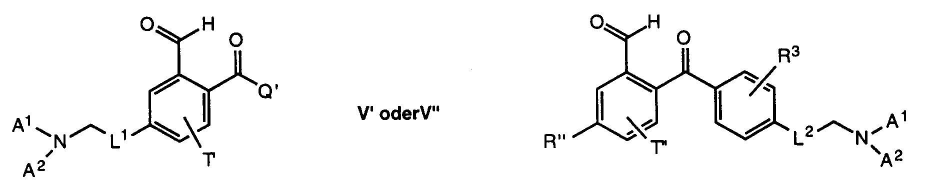 mittels Hydrazin cyclisiert,
e) ein Amin der Formel 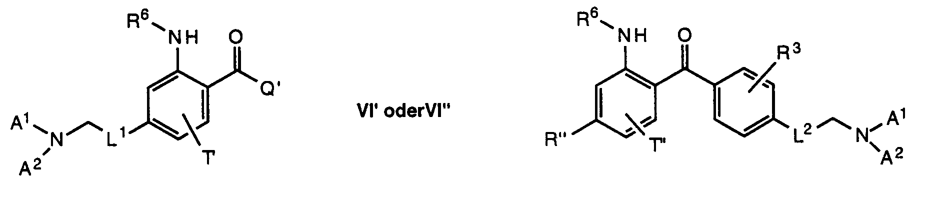 cyclisiert,
f) ein Phenol der Formel 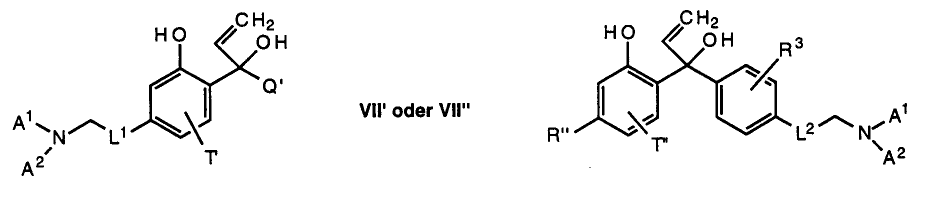 oder ein Anilin der Formel 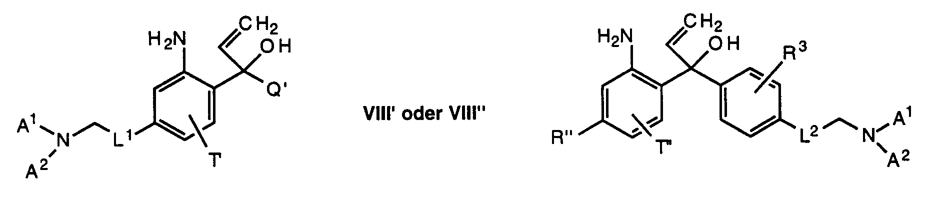 cyclisiert,
g) ein Phenol der Formel 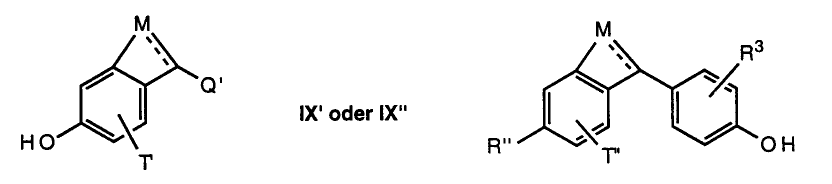 mit einem Aminoalkohol (A¹,A²)NCH₂-L-OH umsetzt,
h) gewünschtenfalls eine in einer Verbindung der Formel I enthaltene reaktionsfähige Gruppe funktionell abwandelt und
i) gewünschtenfalls ein Amin der Formel I in ein physiologisch verträgliches Säureadditionssalz oder ein Säureadditionssalz einer Verbindung der Formel I in das Amin der Formel I überführt.

21. Antimykotisch wirksame und Cholesterin senkende Mittel, enthaltend eine Verbindung nach einem der Ansprüche 1-18 als Wirkstoff gegebenenfalls neben einem therapeutisch inerten Träger.

22. Verwendung der Verbindungen nach einem der Ansprüche 1-18 für die Herstellung von antimykotisch wirksamen und Cholesterin senkenden Mittel.
